# EUROPEAN PATENT APPLICATION

(11) **EP 4 691 357 A1**
(43) Date of publication of application: **11.02.2026**
(21) Application number: 24802501.7
(22) Date of filing: 05.01.2024
(51) Int. Cl.: A61B 5/0235, F16K 31/06

(54) **ELECTROMAGNETIC VALVE AND WEARABLE DEVICE**

(30) Priority: 11.05.2023 CN 202310532060
(71) Applicant: Huawei Technologies Co., Ltd., Shenzhen, Guangdong 518129 (CN)
(72) Inventor: LIN, Yichao, Shenzhen, Guangdong 518129 (CN); WANG, Youhua, Shenzhen, Guangdong 518129 (CN); CHEN, Zhiyuan, Shenzhen, Guangdong 518129 (CN); LI, Changming, Shenzhen, Guangdong 518129 (CN); GONG, Lexing, Shenzhen, Guangdong 518129 (CN); HAN, Suqi, Shenzhen, Guangdong 518129 (CN)
(74) Representative: Isarpatent
(86) International application number: PCT/CN2024/070755
(87) International publication number: WO 2024/230212

(57) **Abstract**

A solenoid valve (4015, 500, 600) and a wearable device are provided. The solenoid valve (4015, 500, 600) includes a housing (1), an electromagnetic component (2), a valve plug component (4), and a reset component (3). The electromagnetic component (2) includes a magnetic yoke (21) and a coil (22). A second medium flow channel (72) and a first accommodation space (213) configured to accommodate the coil (22) are disposed on the magnetic yoke (21). The electromagnetic component (2) and the housing (1) are fastened to form an accommodation cavity configured to accommodate the valve plug component (4) and the reset component (3). The valve plug component (4) is disposed between the electromagnetic component (2) and the housing (1). The reset component (3) is disposed on a side that is of the valve plug component (4) and that faces the electromagnetic component (2) and/or a side that is of the valve plug component (4) and that is away from the electromagnetic component (2). A first medium flow channel (71) and the second medium flow channel (72) are respectively disposed on the housing (1) and the magnetic yoke (21), and the electromagnetic component (2) and the reset component (3) are respectively configured to drive the valve plug component (4) to move when the coil (22) is powered on and is powered off, to enable the first medium flow channel (71) to communicate with or be isolated from the second medium flow channel (72). In the foregoing technical solution, a size of the solenoid valve (4015, 500, 600) can be reduced, to meet a space requirement of an electronic device, achieve miniaturization of the electronic device, and further improve sealability of the solenoid valve (4015, 500, 600).

## Description

This application claims priority to Chinese Patent Application No. 202310532060.7, filed with the China National Intellectual Property Administration on May 11, 2023 and entitled "SOLENOID VALVE AND WEARABLE DEVICE", which is incorporated herein by reference in its entirety.

### TECHNICAL FIELD

Embodiments of this application relate to the field of electronic device technologies, and more specifically, to a solenoid valve and a wearable device.

### BACKGROUND

Blood pressure is an important physiological indicator for monitoring human health, and can reflect a health status of a human body. An electronic sphygmomanometer is a medical device to measure blood pressure based on an electronic technology and an indirect blood pressure measurement principle. Miniaturization of the electronic sphygmomanometer increases portability of the product, so that the electronic sphygmomanometer can be suitable for home use and meet daily blood pressure measurement requirements of families.

With continuous improvement of living standards, people pay more attention to their own health, and require that blood pressure can be dynamically measured anytime and anywhere. Therefore, the electronic sphygmomanometer is evolved to be minimized and wearable.

The electronic sphygmomanometer generally measures blood pressure through airbag inflation and deflation. A solenoid valve is a key accessory for controlling connection and disconnection of an air path of the electronic sphygmomanometer. In an existing electronic sphygmomanometer, to meet a sealability requirement, a size of a solenoid valve is large, and it is difficult for the solenoid valve to meet an extreme space requirement of the electronic sphygmomanometer evolving to be minimized and wearable.

### SUMMARY

Embodiments of this application provide a solenoid valve and a wearable device, to simplify a structure of the solenoid valve and achieve miniaturization of the solenoid valve.

According to a first aspect, a solenoid valve is provided, including: a housing, where a first medium flow channel is disposed on the housing; an electromagnetic component, including a magnetic yoke and a coil, where a second medium flow channel and a first accommodation space that are separated from each other are disposed on the magnetic yoke, the coil is accommodated in the first accommodation space, and the electromagnetic component and the housing are fastened to form an accommodation cavity; a valve plug component, accommodated in the accommodation cavity, where the valve plug component is disposed between the electromagnetic component and the housing; and a reset component, accommodated in the accommodation cavity, where the reset component is disposed on a side that is of the valve plug component and that faces the electromagnetic component and/or a side that is of the valve plug component and that is away from the electromagnetic component. The electromagnetic component is configured to drive the valve plug component to move when the coil is powered on, and the reset component is configured to drive the valve plug component to move when the coil is powered off, to enable the first medium flow channel to communicate with or be isolated from the second medium flow channel.

In embodiments of this application, the magnetic yoke is actually used as a part of an outer housing of the solenoid valve. The electromagnetic component including the magnetic yoke can be fastened to the housing to form the accommodation space for accommodating another component. The solenoid valve component is simplified. This helps reduce a size of the entire solenoid valve, to meet a space requirement of an electronic device, and achieve miniaturization of the electronic device. In addition, the medium flow channels are disposed on the housing and the magnetic yoke. This facilitates overall sealing, to improve air tightness of the solenoid valve, and correspondingly improve anti-corrosion performance and reliability.

With reference to the first aspect, in a possible implementation, a first groove is disposed on a side that is of the housing and that faces the magnetic yoke. The magnetic yoke includes a first body and a protrusion protruding from a surface of the first body, the first body is connected to the housing, the first accommodation space is disposed on the protrusion, and the protrusion is accommodated in the first groove.

The solenoid valve according to embodiments of this application has a simple structure, and is the simplified component, so that a manufacturing process is simpler and more reliable, and dimensional precision of a component of the solenoid valve can be improved, thereby facilitating assembly of the solenoid valve and improving a yield rate.

In addition, the first accommodation space for accommodating the coil is disposed on the magnetic yoke, so that a single component encloses the coil, and the magnetic yoke does not need to cooperate with another component to form a mounting groove for accommodating the coil. This facilitates mounting of the coil, the assembly of the solenoid valve, and the miniaturization of the solenoid valve, effectively saves space, facilitates overall sealing, and improves sealability of the solenoid valve. In addition, the magnetic yoke can transmit a magnetic line generated by the power-on coil to a required place, to form a magnetic field at the required place, thereby reducing magnetic leakage.

With reference to the first aspect, in a possible implementation, the first medium flow channel communicates with the first groove; and the second medium flow channel communicates with the first groove.

The first medium flow channel and the second medium flow channel are respectively disposed on the housing and the magnetic yoke, and the first medium flow channel can communicate with the second medium flow channel via the space formed by fastening the housing and the electromagnetic component. Because the accommodation cavity is formed by a connection between the housing and the electromagnetic component, there are a few sealing interfaces, and sealing reliability is improved. As a result, sealability of an entire path is good.

With reference to the first aspect, in a possible implementation, the first medium flow channel includes a first opening and a second opening, the first opening is disposed on a bottom wall or a side wall of the first groove, and the second opening is disposed on an outer wall of the housing; and/or the second medium flow channel includes a third opening and a fourth opening, the third opening is disposed on a surface that is of the protrusion and that faces the bottom wall of the first groove, and the fourth opening is disposed on a surface that is of the first body and that is away from the bottom wall of the first groove.

A single component, namely, the housing, forms the complete first medium flow channel, so that sealability of a pipe of the solenoid valve can be improved, and the assembly can be facilitated.

A single component, namely, the magnetic yoke, forms the complete second medium flow channel, so that sealability of a pipe of the solenoid valve can be improved, and the assembly can be facilitated.

With reference to the first aspect, in a possible implementation, a center line of the first medium flow channel is a straight line; and/or a center line of the second medium flow channel is a straight line.

In this way, the first medium flow channel or the second medium flow channel has little resistance to an operating medium (such as gas or liquid) of the solenoid valve, so that the operating medium can smoothly pass through the first medium flow channel and the second medium flow channel, thereby improving a response speed and pressure relief efficiency of the solenoid valve, and shortening pressure relief time.

With reference to the first aspect, in a possible implementation, the center line of the first medium flow channel coincides with the center line of the second medium flow channel.

In this way, during pressure relief, the operating medium of the solenoid valve can smoothly reach an outlet from an inlet of the solenoid valve, a flow distance is short, the resistance is low, the response speed and the pressure relief efficiency of the solenoid valve are improved, and the pressure relief time is shortened.

With reference to the first aspect, in a possible implementation, the first accommodation space is disposed around the second medium flow channel.

In this way, it is convenient to design a space (namely, the first accommodation space) for accommodating the coil and the second medium flow channel on the magnetic yoke.

With reference to the first aspect, in a possible implementation, the protrusion includes a first protrusion and a second protrusion, the second protrusion is disposed around the first protrusion, and the first accommodation space is formed between the first protrusion and the second protrusion.

In this way, the magnetic yoke is easy to process and the coil is easy to mount. In addition, when a single coil is disposed in the first accommodation space, a circuit connection and a cabling layout can be simplified.

With reference to the first aspect, in a possible implementation, an opening that is of the second medium flow channel and that is close to the valve plug component is disposed on a surface that is of the first protrusion and that faces the bottom wall of the first groove.

In this way, the opening that is of the second medium flow channel and that is close to the valve plug component is basically at a middle location, and the valve plug component is evenly supported by a force, so that blocking effect of the valve plug component can be improved.

With reference to the first aspect, in a possible implementation, the reset component includes an elastic member, the elastic member is sleeved on the protrusion, one end of the elastic member presses against the valve plug component, and the other end of the elastic member presses against the first body.

The elastic member has a good elastic deformation capability, is easy to process and arrange, and can provide high reliability.

With reference to the first aspect, in a possible implementation, the elastic member is any one of a spring, a corrugated pipe, and an elastic block.

With reference to the first aspect, in a possible implementation, when the coil is powered on, the magnetic yoke attracts the valve plug component, the elastic member is in a compressed state, and the valve plug component abuts against the protrusion under action of a magnetic force of the magnetic yoke and an elastic force of the elastic member.

With reference to the first aspect, in a possible implementation, the reset component includes a magnetic member, the magnetic member is accommodated in a second accommodation space disposed on the housing, the second accommodation space is close to the bottom wall of the first groove and corresponds to a location of the valve plug component, the magnetic member and the coil are respectively disposed on two sides of the valve plug component, and a magnetic attraction force exists between the magnetic member and the valve plug component.

The valve plug component moves under action of a magnetic force of the magnetic member, so that the valve plug component can be more evenly supported by the force, and moves more smoothly, thereby avoiding tilting and unsmooth movement.

With reference to the first aspect, in a possible implementation, the magnetic member is injected into the housing by using an insert molding process.

An integrated product made in this way avoids an addition of a new sealing interface. This helps improve the sealability of the solenoid valve, and does not affect performance of the magnetic member.

With reference to the first aspect, in a possible implementation, the magnetic member is a permanent magnet or a soft magnet.

With reference to the first aspect, in a possible implementation, when the coil is powered on, the magnetic yoke attracts the valve plug component, and the valve plug component abuts against the protrusion under action of the magnetic force of the magnetic yoke and the magnetic force of the magnetic member.

With reference to the first aspect, in a possible implementation, when the coil is powered on, the valve plug component blocks the second medium flow channel, and when the coil is not powered on, the first medium flow channel communicates with the second medium flow channel via the first groove; or when the coil is not powered on, the valve plug component blocks the first medium flow channel, and when the coil is powered on, the first medium flow channel communicates with the second medium flow channel via the first groove.

With reference to the first aspect, in a possible implementation, the valve plug component includes a sealing member and a separator, and the separator is configured to drive, under driving of the electromagnetic component or the reset component, the sealing member to move, to block the first medium flow channel or the second medium flow channel.

With reference to the first aspect, in a possible implementation, an end that is of the sealing member and that is close to the first medium flow channel protrudes towards the first medium flow channel relative to the separator, and/or an end that is of the sealing member and that is close to the second medium flow channel protrudes towards the second medium flow channel relative to the separator.

A protruding part of the sealing member can ensure enough deformation, and can be configured to form good sealing at a blocking location.

With reference to the first aspect, in a possible implementation, a second groove that communicates with the second medium flow channel is disposed on a side that is of the protrusion and that faces the bottom wall of the first groove, and the second groove is configured to accommodate a part that is of the sealing member and that protrudes towards the second medium flow channel relative to the separator; and/or a third groove that communicates with the first medium flow channel is disposed on the bottom wall of the first groove, and the third groove is configured to accommodate a part that is of the sealing member and that protrudes towards the first medium flow channel relative to the separator.

In this way, thickness of the solenoid valve in a direction in which the housing is fastened to the electromagnetic component can be reduced while sealability of the sealing member is met, thereby helping achieve the miniaturization of the solenoid valve.

With reference to the first aspect, in a possible implementation, the sealing member is configured to block the first medium flow channel. When the coil is powered on, the part that is of the sealing member and that protrudes towards the second medium flow channel relative to the separator is accommodated in the second medium flow channel, and a gap exists between an inner wall of the second medium flow channel and the sealing member. Alternatively, the sealing member is configured to block the second medium flow channel. When the coil is not powered on, the part that is of the sealing member and that protrudes towards the first medium flow channel relative to the separator is accommodated in the first medium flow channel, and a gap exists between an inner wall of the first medium flow channel and the sealing member.

In this way, thickness of the solenoid valve in a direction in which the housing is fastened to the magnetic yoke can be reduced while the first medium flow channel can communicate with the second medium flow channel, thereby helping achieve the miniaturization of the solenoid valve.

With reference to the first aspect, in a possible implementation, the separator includes a permanent magnet material or a soft magnetic material.

With reference to the first aspect, in a possible implementation, at least one through hole is disposed on the separator, and the through hole is configured to communicate the first medium flow channel with the second medium flow channel.

With reference to the first aspect, in a possible implementation, a protruding part is disposed on the bottom wall of the first groove or on a surface that is of the separator and that faces the bottom wall of the first groove, the separator and the bottom wall of the first groove abut against each other via the protruding part, the protruding part and the through hole are disposed in a staggered manner, and a gap is formed in a region in which the protruding part is not disposed between the bottom wall of the first groove and the separator.

The gap formed between the separator and the bottom wall of the first groove due to disposing of the protruding part can provide enough space for the operating medium to flow when the solenoid valve is in an open state, thereby facilitating quick pressure relief.

With reference to the first aspect, in a possible implementation, the solenoid valve further includes a dustproof component, and the dustproof component is disposed on the first medium flow channel and/or the second medium flow channel of the solenoid valve.

Herein, one of the first medium flow channel and the second medium flow channel is an inlet flow channel of the solenoid valve, and the other is an outlet flow channel of the solenoid valve. In other words, the dustproof component may be disposed at the inlet flow channel and/or the outlet flow channel of the solenoid valve. The dustproof component can prevent foreign objects such as dust from entering the solenoid valve, to avoid affecting a normal function of the solenoid valve.

With reference to the first aspect, in a possible implementation, the solenoid valve further includes an electrical connector, the electrical connector is disposed on the outer wall of the housing, and the electrical connector is electrically connected to the coil via a cable hole disposed on the magnetic yoke.

With reference to the first aspect, in a possible implementation, the housing is of an integral structure, and/or the magnetic yoke is of an integral structure. The integrated housing and/or magnetic yoke can simplify a processing process.

With reference to the first aspect, in a possible implementation, the housing is in sealing connection to the magnetic yoke. In this way, it is beneficial to implement overall sealing of the solenoid valve.

With reference to the first aspect, in a possible implementation, the solenoid valve further includes a substrate, the substrate is connected to the housing or the magnetic yoke, a third medium flow channel is disposed on the substrate, and the third medium flow channel communicates with a non-outlet flow channel of the first medium flow channel and the second medium flow channel.

A single component, namely, the substrate, can form the complete third medium flow channel, so that sealability of a pipe can be improved, and the assembly can be facilitated. In addition, the first medium flow channel, the second medium flow channel, and the third medium flow channel are all flow channels disposed inside the components, and their paths can be designed based on an actual requirement, eliminating the need for piping layout via a sleeve at the solenoid valve, so that the solenoid valve can have a more uniform shape, and an overall size can be reduced, to be applicable to an extreme space requirement of an electronic device such as a wearable product.

With reference to the first aspect, in a possible implementation, the operating medium of the solenoid valve is the gas or the liquid.

According to a second aspect, a component for pressurization and pressure relief are provided, including the solenoid valve according to any one of the first aspect or the possible implementations of the first aspect.

According to a third aspect, a wearable device is provided, including an airbag and the solenoid valve according to any one of the first aspect or the possible implementations of the first aspect. The airbag communicates with the solenoid valve. When the airbag is inflated, the first medium flow channel of the solenoid valve is isolated from the second medium flow channel; and when the airbag is deflated, the first medium flow channel of the solenoid valve communicates with the second medium flow channel.

The solenoid valve according to embodiments of this application has good air tightness. When the solenoid valve is used in a wearable device, accuracy of a parameter measured via the airbag can be improved.

With reference to the third aspect, in a possible implementation, the wearable device further includes a binding band, and the binding band is configured to bind the airbag to a body part of a user.

With reference to the third aspect, in a possible implementation, the airbag is packaged in the binding band.

With reference to the third aspect, in a possible implementation, the body part of the user includes any one of a wrist, an arm, and an ankle.

With reference to the third aspect, in a possible implementation, the wearable device further includes an air pump, and the air pump is configured to inflate the airbag.

With reference to the third aspect, in a possible implementation, the wearable device further includes a pressure sensor, and the pressure sensor is configured to detect pressure of the airbag.

With reference to the third aspect, in a possible implementation, the wearable device is an electronic sphygmomanometer. For example, the wearable device is a blood pressure watch or a blood pressure band.

When the solenoid valve according to embodiments of this application is used in the electronic sphygmomanometer, miniaturization of the electronic sphygmomanometer is facilitated, and accuracy of blood pressure measurement can be further improved.

For beneficial effect of the apparatuses according to the second aspect and the third aspect, refer to the related descriptions in the first aspect. For brevity, details are not described again.

### BRIEF DESCRIPTION OF DRAWINGS

FIG. 1 is a diagram of scenarios to which an embodiment of this application is applicable;
FIG. 2 is a diagram of structures of electronic devices according to an embodiment of this application;
FIG. 3 is an assembly diagram of a solenoid valve according to an embodiment of this application;
FIG. 4 is an exploded diagram of a solenoid valve according to an embodiment of this application;
FIG. 5 is a cross-sectional diagram of a solenoid valve according to an embodiment of this application;
FIG. 6 is a cross-sectional diagram of another solenoid valve according to an embodiment of this application;
FIG. 7 is an exploded diagram of a solenoid valve according to an embodiment of this application;
FIG. 8 is a cross-sectional diagram of a solenoid valve according to an embodiment of this application;
FIG. 9 is a cross-sectional diagram of another solenoid valve according to an embodiment of this application;
FIG. 10 is an assembly diagram of a component for pressurization and pressure relief according to an embodiment of this application;
FIG. 11 is an exploded diagram of a component for pressurization and pressure relief according to an embodiment of this application; and
FIG. 12 is a cross-sectional diagram of a component for pressurization and pressure relief according to an embodiment of this application.

### DESCRIPTION OF EMBODIMENTS

The following describes technical solutions of embodiments in this application with reference to accompanying drawings.

It should be noted that, in descriptions of embodiments of this application, unless otherwise specified, "/" represents an "or" relationship. For example, A/B may represent A or B. "And/or" in this specification is merely an association relationship for describing associated objects, and represents that three relationships may exist. For example, A and/or B may represent the following three cases: Only A exists, both A and B exist, and only B exists.

The terms "first" and "second" in embodiments of this application are merely intended for a purpose of description, and shall not be understood as an indication or implication of relative importance or implicit indication of a quantity of indicated technical features. Therefore, a feature limited by "first" or "second" may explicitly or implicitly include one or more features. In addition, in the descriptions of embodiments of this application, "a plurality of" means two or more, and "at least one" and "one or more" mean one, two, or more. The singular expression forms "one", "a", "the", "the foregoing", "this", and "the one" are also intended to include expression forms such as "one or more", unless otherwise specified in the context clearly.

Reference to "an embodiment", "some embodiments", or the like described in this specification means that one or more embodiments of this application include a specific feature, structure, or characteristic described with reference to embodiments. Therefore, statements such as "in an embodiment", "in some embodiments", "in some other embodiments", and "in other embodiments", that appear at different places in this specification do not necessarily mean referring to a same embodiment, instead, they mean "one or more but not all of embodiments", unless otherwise specifically emphasized. The terms "include", "contain", "have", and their variants all mean "include but are not limited to", unless otherwise specifically emphasized.

In the descriptions of embodiments of this application, orientations or location relationships indicated by terms such as "up", "down", "left", "right", "inner", "outer", "vertical", and "horizontal" are defined relative to orientations or locations of components schematically placed in the accompanying drawings. It should be understood that these orientation terms are relative concepts, and are used for relative description and clarification, instead of indicating or implying that specified apparatuses or components need to have specific orientations or are constructed and operated in specific orientations. The terms may vary correspondingly based on changes of the orientations of the components placed in the accompanying drawings, and therefore shall not be understood as a limitation on this application. In addition, "vertical" in this application is not strictly vertical, but is vertical within an allowed error range. "Parallel" is not strictly parallel, but within an allowable error range.

In embodiments of this application, same reference numerals indicate same components or parts. For same components or parts in embodiments of this application, only one component or part may be marked with a reference numeral in the figure as an example. It should be understood that, this is also applicable to reference numerals of other same components or parts. In addition, the accompanying drawings are not drawn to actual scale. Dimensions and sizes of components or parts shown in the figures are merely examples, and should not be understood as a limitation on this application.

For ease of understanding, the following first explains and describes technical terms in this application.

A magnetic yoke is usually a soft magnetic material that does not produce a magnetic field (magnetic field line) and transmits only the magnetic field line in a magnetic circuit. Magnetic yokes are generally made of soft iron, A3 steel (namely, Q235 carbon structural steel), and soft magnetic alloys with high magnetic permeability. In some special cases, magnetic yokes can alternatively be made of ferrite materials. The magnetic yoke can have the following functions: preventing magnetic leakage of an induction coil from spreading to the outside, improving efficiency of induction heating, supporting and fastening the induction coil, and the like.

Magnetic effect of a current is a phenomenon that any wire with a current can generate a magnetic field around the wire. For example, a coil is powered on to generate a magnetic field because a current magnetizes the coil.

An elastic member is an element with elastic performance, that is, an element that can deform under action of an external force and can restore to an original state after the external force is removed.

A permanent magnet is a magnet that can maintain magnetism for long time. The permanent magnet is a hard magnet, which is difficult to demagnetize and magnetize.

A permanent magnet material is a material that is difficult to magnetize and is difficult to demagnetize after the material is magnetized. A main feature of the permanent magnet material is that the permanent magnet material has a high coercive force (usually greater than 1000 ampere per meter (A/m)).

A soft magnet is a magnet that is easy to magnetize, and whose magnetism easily disappears after the magnet is magnetized and cannot be maintained for long time.

A soft magnetic material is a magnetic material having a low coercive force (less than 1000 A/m, and usually less than 100 A/m) and high magnetic permeability. A main feature of the soft magnetic material is that the soft magnetic material is easy to magnetize and demagnetize, and largest magnetization can be achieved based on a smallest external magnetic field.

A coercive force (coercive force) is a magnetic field strength in which a magnetic material is magnetized and then demagnetized to reduce remanent magnetism (remanent flux density or remanent magnetization) of the magnetic material to zero. The coercive force is also referred to as a coercive magnetic field, indicated by a symbol Hc. Generally, remanent magnetism of a soft magnet is small or very small, and remanent magnetism of a permanent magnet is large. Therefore, a coercive force of the permanent magnet is greater than a coercive force of the soft magnet.

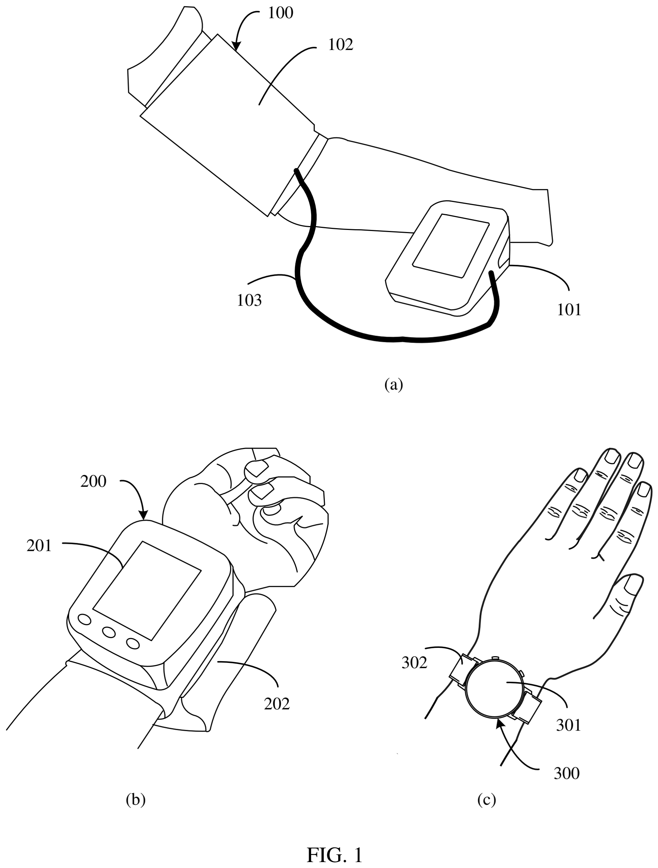

With continuous improvement of living standards, people pay more attention to their own health. Blood pressure, as a main physiological indicator of a human body, is also increasingly valued by people. An electronic sphygmomanometer is a medical device to measure blood pressure based on an electronic technology and an indirect blood pressure measurement principle. Miniaturization of the electronic sphygmomanometer increases portability of the product, so that the electronic sphygmomanometer can be suitable for home use and meet daily blood pressure measurement requirements of families. Currently, product forms of electronic sphygmomanometers on the market mainly include an arm electronic sphygmomanometer (which is referred to as an arm sphygmomanometer for short in the following embodiments), a wrist electronic sphygmomanometer (which is referred to as a wrist sphygmomanometer for short in the following embodiments), and a watch electronic sphygmomanometer (which is referred to as a watch sphygmomanometer or a blood pressure watch for short in the following embodiments). For example, FIG. 1 shows diagrams of the foregoing several types of electronic sphygmomanometers.

(a) in FIG. 1 is a diagram of a use scenario of an arm sphygmomanometer. As shown in (a) in FIG. 1, the arm sphygmomanometer 100 may include a main unit 101, a cuffband 102, and a gas pipe 103. An air pump is disposed in the main unit 101, an airbag is packaged in the cuffband 102, and the gas pipe 103 is connected to the air pump in the main unit 101 and the airbag in the cuffband 102. During use, the cuffband 102 may be wound around and bound to an arm of a human body. The main unit 101 can control the air pump to inflate the airbag, so that the airbag expands and presses blood vessels, and the main unit 101 can control airbag to deflate, thereby measuring blood pressure.

(b) in FIG. 1 is a diagram of a use scenario of a wrist sphygmomanometer. As shown in (b) in FIG. 1, the wrist sphygmomanometer 200 may include a main unit 201 and a wristband 202. An air pump is disposed in the main unit 201, an airbag is packaged in the wristband 202, and the air pump communicates with the airbag. During use, the wristband 202 may be wound around and bound to a wrist of a human body. The main unit 201 is located on a side of a palm. The main unit 201 can control the air pump to inflate the airbag, so that the airbag expands and presses blood vessels, and the main unit 201 can control airbag to deflate, thereby measuring blood pressure.

(c) in FIG. 1 is a diagram of a use scenario of a watch sphygmomanometer. As shown in (c) in FIG. 1, the watch sphygmomanometer 300 may include a main unit 301 and a watchband 302. An air pump is disposed in the main unit 301, an airbag is packaged in the watchband 302, and the air pump communicates with the airbag. During use, the watchband 302 may be wound around and bound to a wrist of a human body. The main unit 301 is located on a side of the back of a hand. The main unit 301 can control the air pump to inflate the airbag, so that the airbag expands and presses blood vessels, and the main unit 301 can control airbag to deflate, thereby measuring blood pressure. Certainly, in another embodiment, in addition to the watch sphygmomanometer, the electronic sphygmomanometer in this application may alternatively be another wearable sphygmomanometer, for example, a portable sphygmomanometer suitable for long-term wearing such as a blood pressure band.

Most electronic sphygmomanometers use oscillometric methods to indirectly measure blood pressure. The arm sphygmomanometer 100 shown in (a) in FIG. 1 is used as an example. A process of measuring blood pressure by using an oscillometric method is as follows: After the cuffband 102 is bound, the main unit 101 can control the air pump to inflate the airbag, so that the airbag expands and presses the blood vessels. When expanding to a given extent, the airbag presses the blood vessels to occlude, and stops blood flow. When the blood flow is completely stopped, the main unit 101 controls the airbag to deflate. In this case, the blood vessels generate vibration waveforms. The vibration waveform can cause gas oscillation in the airbag, and an oscillation waveform of gas is related to the vibration waveforms of the blood vessels. The main unit 101 can collect an oscillation waveform signal of the gas, and process the oscillation waveform signal according to a built-in algorithm (for example, an amplitude coefficient method), to calculate a blood pressure value. Blood pressure measurement principles of the wrist sphygmomanometer 200 and the watch sphygmomanometer 300 are the same as those described above. For brevity, details are not described again.

It may be understood that the types and the measurement principles of the electronic sphygmomanometer described above are merely examples. In some other embodiments, the electronic sphygmomanometer may further have another product form (for example, the blood pressure band), or may use another measurement principle (for example, a Korotkoff sounds method). Details are not described herein.

The technical solutions according to embodiments of this application may be applied to an electronic device having functions of pressurization and pressure relief, for example, an electronic device having a blood pressure monitoring function. In some embodiments, the electronic device may be a wearable device. The wearable device may be a portable device that can be integrated into clothes or accessories of a user, has a computing function, and may further be connected to a mobile phone or another terminal device. For example, the wearable device may be a smartwatch (as shown in (c) in FIG. 1), a smart band, the wrist sphygmomanometer (as shown in (b) in FIG. 1), the arm sphygmomanometer (as shown in (a) in FIG. 1), or the like. A type of the wearable device is not specifically limited in this application.

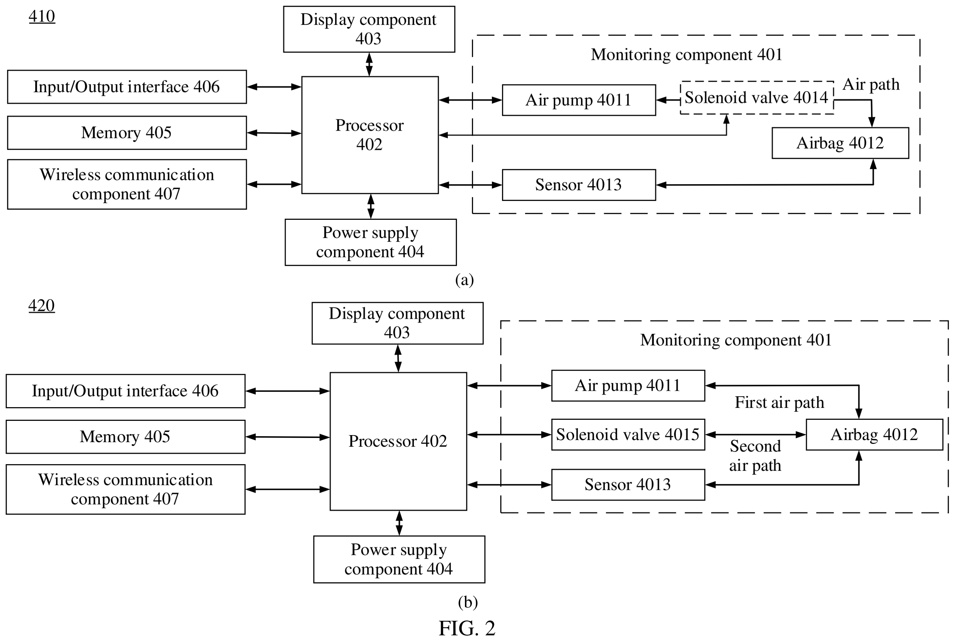

FIG. 2 is a diagram of structures of electronic devices according to an embodiment of this application. For example, an electronic device 410 shown in (a) in FIG. 2 or an electronic device 420 shown in (b) in FIG. 2 may be a specific example of the electronic sphygmomanometer (for example, the arm sphygmomanometer 100, the wrist sphygmomanometer 200, or the watch sphygmomanometer 300) shown in FIG. 1. In other words, the electronic device 410 or the electronic device 420 may have a blood pressure monitoring function.

As shown in (a) in FIG. 2 or (b) in FIG. 2, the electronic device 410 or the electronic device 420 may include a monitoring component 401, a processor 402, a display component 403, a power supply component 404, a memory 405, and an input/output interface 406. In some embodiments, the electronic device 410 or the electronic device 420 may further include a wireless communication component 407.

It should be noted that a main difference between the electronic device 410 shown in (a) in FIG. 2 and the electronic device 420 shown in (b) in FIG. 2 lies in that monitoring components 401 are slightly different. The following provides detailed descriptions.

The monitoring component 401 is a core component configured to monitor blood pressure. For example, as shown in (a) in FIG. 2, in the electronic device 410, the monitoring component 401 may include an air pump 4011, an airbag 4012, and a sensor 4013.

The air pump 4011 communicates with the airbag 4012, and is configured to inflate the airbag 4012 or deflate the airbag 4012 (that is, discharge air in the airbag 4012). For example, when the air pump 4011 inflates the airbag 4012, an air outlet disposed in the air pump 4011 is in a closed state, and pressure of the airbag 4012 continuously increases. When gas in the airbag 4012 needs to be discharged, the air outlet disposed in the air pump 4011 is in an open state, and the gas is discharged through the air outlet. In this embodiment of this application, the air pump 4011 may be an electric air pump, a hand-operated air pump, or a foot-operated air pump. For example, the air pump 4011 may be a miniature air pump, for example, a miniature diaphragm air pump, a miniature electromagnetic air pump, a miniature impeller air pump, or a miniature piston micro air pump. A specific type of the air pump 4011 is not limited in this application.

The airbag 4012 is configured to store air filled by the air pump 4011, and may be attached to a to-be-monitored part of a user during use. For example, the electronic device 410 may further include a binding band, and the airbag 4012 is packaged in the binding band. When the electronic device 410 is used, the airbag 4012 may be surrounded and attached to the to-be-monitored part of the user via the binding band. For example, the to-be-monitored part of the user may be a wrist, an upper arm, a foot wrist, or another body part of the user. Correspondingly, the binding band may also have a corresponding name, for example, a cuffband, a wristband, a watchband, or an ankleband. This is not limited in embodiments of this application.

The sensor 4013 is connected to the airbag 4012, and is configured to obtain a sensor parameter to calculate a blood pressure value. For example, the sensor 4013 may include a pressure sensor and/or a pulse wave signal sensor. The pressure sensor (for example, a barometer) is configured to detect atmospheric pressure of the airbag 4012, and the pulse wave signal sensor is configured to measure a pulse wave signal. By way of example rather than limitation, in an example of measuring blood pressure by using an oscillometric method, the sensor 4013 may use the barometer. The barometer can collect an oscillation waveform signal of the gas through an air path between the barometer and the airbag 4012, and send the oscillation waveform signal to the processor 402.

In some embodiments, the sensor 4013 may be connected to the airbag 4012 through the air path, and the sensor 4013 measures the atmospheric pressure of the airbag 4012 through the air path. In some other embodiments, the sensor 4013 may be attached to an inner wall of the airbag 4012 to measure the atmospheric pressure of the airbag 4012.

In some embodiments, a solenoid valve 4014 may be disposed in the air path between the air pump 4011 and the airbag 4012, to control connection and disconnection of the air path between the air pump 4011 and the airbag 4012, thereby inflating and/or deflating the airbag 4012. For example, when the airbag 4012 needs to be inflated, the solenoid valve 4014 is in an open state, and the gas may be transferred to the airbag 4012 by the air pump 4011. When the gas in the airbag 4012 needs to be discharged, the solenoid valve 4014 is in the open state, and the gas may flow from the airbag 4012 to the air pump 4011. In addition, when the pressure of the airbag 4012 needs to be maintained, the solenoid valve 4014 may be in a closed state.

For another example, as shown in (b) in FIG. 2, in the electronic device 420, the monitoring component 401 may include the air pump 4011, the airbag 4012, the sensor 4013, and a solenoid valve 4015. The air pump 4011 and the solenoid valve 4015 are respectively connected to air paths of the airbag 4012, and are used as two air outlets of the airbag 4012. The air path between the air pump 4011 and the airbag 4012 may be referred to as a first air path, and an air path between the solenoid valve 4015 and the airbag 4012 may be referred to as a second air path. In some embodiments, the solenoid valve 4015 may also be referred to as a pressure relief valve.

Herein, when both the first air path and the second air path are disposed, the first air path may be used to inflate the airbag 4012 or inflate and deflate the airbag 4012, and the second air path may be used to deflate the airbag 4012. In other words, the airbag 4012 may be inflated via the air path connected to the air pump 4011, and the airbag 4012 may be deflated via one or more air paths. For example, the airbag 4012 may be deflated via the first air path and the second air path. An air inlet and an air outlet of the first air path are respectively the airbag 4012 and the air pump 4011, and an air inlet and an air outlet of the second air path are respectively the airbag 4012 and the solenoid valve 4015. In other words, the gas in the airbag 4012 may be discharged through two different air outlets. For example, when the airbag 4012 needs to be inflated, the air outlet disposed in the air pump 4011 is in the closed state, the solenoid valve 4015 is in the open state, and the air pump 4011 may fill the airbag 4012 with the gas. When the gas in the airbag 4012 needs to be discharged, the air outlet of the air pump 4011 is in the open state, the solenoid valve 4015 is in the open state, and the gas in the airbag 4012 is discharged via the air pump 4011 and the solenoid valve 4015; or the air outlet of the air pump 4011 is in the closed state, the solenoid valve 4015 is in the open state, and the gas in the airbag 4012 is discharged via only the solenoid valve 4015.

It may be understood that, in the electronic device 420 shown in (b) in FIG. 2, the solenoid valve 4014 shown in (a) in FIG. 2 may alternatively be disposed in the first air path. For an operating principle and process of the solenoid valve 4014, refer to the foregoing related description of the solenoid valve 4014. For brevity, details are not described again.

Herein, the first air path and the second air path may be independent of each other and have no overlapping path, or the first air path and the second air path partially overlap. This is not limited in this application. In some embodiments, if two air paths have a same air inlet and different air outlets, or two air paths have different air inlets and a same air outlet, the two air paths may be referred to as side by side or parallel.

In this embodiment of this application, the airbag 4012 is connected to the air pump 4011 through the air path, and the airbag 4012 is connected to the solenoid valve 4015 through the air path. The airbag 4012 and the sensor 4013 may be connected through the air path. During actual application, parts such as the air pump 4011, the airbag 4012, the sensor 4013, and the solenoid valve 4015 that need to be connected through the air path may be implemented via an air path connector.

It may be understood that the sensor 4013 used for blood pressure monitoring is only one type of sensor included in the electronic device 410 or 420. In some other embodiments, the electronic device 410 or 420 may further include another type of sensor, for example, a motion sensor (for example, a gyroscope sensor or an acceleration sensor) configured to obtain motion data or posture data of the user, and a biosensor (for example, an optical heart rate sensor, a blood oxygen sensor, a bioimpedance sensor, an electrocardiography sensor, an electrodermal activity sensor, or a skin temperature sensor) configured to obtain a biosignal of the user. This is not limited in embodiments of this application.

The processor 402 may be configured to: control and process information, connect all parts of an entire electronic device (for example, the electronic device 410 or 420) through various interfaces and lines, and perform various functions of the electronic device and data processing, to perform overall monitoring on operation of the electronic device. In this embodiment of this application, the air pump 4011, the solenoid valve (for example, the solenoid valve 4014 or the solenoid valve 4015), and the sensor 4013 are connected to the processor 402. Under control of the processor 402, the air pump 4011 is configured to inflate the airbag 4012, the solenoid valve is configured to control the connection and disconnection of the air path, and the sensor 4013 is configured to obtain the sensing parameter for calculating the blood pressure value, to implement blood pressure monitoring. By way of example rather than limitation, for example, the processor 402 may be connected to the air pump 4011, to control the air pump 4011 to inflate or deflate the airbag 4012. The processor 402 may alternatively be connected to the sensor 4013, and is configured to obtain a pressure signal of the airbag 4012 detected by the sensor 4013 or the detected pulse wave signal. The processor 402 may alternatively be connected to the solenoid valve 4015, to control the solenoid valve 4015 to deflate the airbag 4012.

The processor 402 may include one or more processing units. For example, the processor 402 may include an application processor (application processor, AP), a graphics processing unit (graphics processing unit, GPU), an image signal processor (image signal processor, ISP), a baseband processor, a modem processor, a controller, and the like. Different processing units may be independent components, or may be integrated into one or more processors. The controller may be a nerve center and a command center of the electronic device. The controller may generate an operation control signal based on instruction operation code and a time sequence signal, to complete control of instruction fetching and instruction execution.

A memory may be further disposed in the processor 402, to store instructions and data. For example, the memory in the processor 402 may be a cache memory. The memory may store instructions or data just used or cyclically used by the processor 402. If the processor 402 needs to use the instructions or the data for another time, the processor 402 may directly invoke the instructions or the data from the memory. This avoids repeated access and reduces waiting time of the processor 402, and improves data processing or instruction execution efficiency of the electronic device.

The display component 403 is configured to display an image or a video, for example, display information entered by the user or information provided for the user, and various menu screens of the electronic device. For example, the display component 403 may display a blood pressure measurement result. The display component 403 includes a display panel. In some embodiments, the display panel may be configured in a form such as a liquid crystal display (liquid crystal display, LCD), an organic light-emitting diode (organic light-emitting diode, OLED), an active-matrix organic light-emitting diode (active-matrix organic light-emitting diode, AMOLED), a flexible light emitting diode (flexible light emitting diode, FLED), quantum dot light emitting diodes (quantum dot light emitting diodes, QLED), or the like.

The power supply component 404 is configured to provide a system power supply of the electronic device, and supply power to the components of the electronic device. The power supply component 404 may support the electronic device to receive charging input. In some embodiments, the power supply component 404 may include a power management unit (power management unit, PMU) and a battery. The power management unit PMU may include a charging circuit, a voltage drop regulation circuit, a protection circuit, a power measurement circuit, and the like. The charging circuit may receive external charging input. The voltage drop regulation circuit may perform voltage regulation on an electrical signal input by the charging circuit, and output a voltage to the battery to complete charging of the battery, or perform voltage regulation on an electrical signal input by the battery, and output a voltage to another component such as the monitoring component 401, the display component 403, and the wireless communication component 407. The protection circuit may be configured to prevent overcharge, overdischarge, short circuit, overcurrent, or the like of the battery. The power management unit may be further configured to monitor a parameter such as battery capacity, a quantity of battery cycles, and a battery health status (electric leakage or impedance).

The memory 405 may be configured to store computer-executable program code. The executable program code includes instructions. The processor 402 runs the instructions stored in the memory 405, to perform various function applications of the electronic device and data processing, for example, implements a charging function, a wireless communication function, and the blood pressure monitoring function of the electronic device.

The input/output interface 406 is configured to provide a wired connection for the electronic device to perform charging or communication. In some embodiments, the input/output interface 406 may include an electrical connector, and the electrical connector is configured to conduct and transmit a current.

The wireless communication component 407 may be configured to support data exchange between the electronic device and another device in wireless communication such as Bluetooth (Bluetooth, BT), a global navigation satellite system (global navigation satellite system, GNSS), a wireless local area network (wireless local area network, WLAN) (for example, a wireless fidelity (wireless fidelity, Wi-Fi) network), frequency modulation (frequency modulation, FM), near field communication (near field communication, NFC), or an infrared (infrared, IR) technology. In some embodiments, the wireless communication component 407 may include a Bluetooth chip. The electronic device may pair with a Bluetooth chip of another electronic device via the Bluetooth chip and set up a wireless connection, to implement wireless communication between the electronic device and the another device through the wireless connection. The wireless communication component 407 may be one or more components that integrate at least one communication processor module.

In some embodiments, the electronic device shown in (a) or (b) in FIG. 2 may further include an audio component, so that the electronic device inputs and outputs an audio signal.

In some embodiments, in the structure shown in FIG. 2, in addition to the airbag 4012, other components or modules may be integrated into the main unit in FIG. 1, for example, the main unit 101, the main unit 201, or the main unit 301.

It may be understood that an example structure in embodiments of this application does not constitute a specific limitation on the electronic device 410 or 420. In some other embodiments of this application, the electronic device 410 or 420 may include more or fewer components than those shown in the figure, or some components may be combined, or some components may be split, or different component arrangements may be used. The components shown in the figure may be implemented by using hardware, software, or a combination of software and hardware.

In addition, it should be noted that during blood pressure measurement, in addition to that pressure is applied to the to-be-monitored part of the user through the gas, the pressure may be applied to the to-be-monitored part of the user through liquid. In other words, in some embodiments, the airbag 4012 may be replaced with a flexible component that can be filled with liquid. In this case, the monitoring component 401 may not include the air pump 4011.

For ease of description and understanding, in embodiments of this application, components that apply pressure to the to-be-monitored part of the user are collectively referred to as a pressure bag. The pressure bag may be filled with gas or liquid. Correspondingly, in the diagram of the structure shown in FIG. 2, the sensor 4013 may be a pressure sensor that can measure pressure of the pressure bag or the pulse wave signal sensor that can measure the pulse wave signal, and the solenoid valve 4015 may be a pressure relief valve that can discharge the gas or the liquid in the pressure bag. For ease of understanding, the following describes the technical solutions according to embodiments of this application by using an example in which the pressure bag is the airbag. In some other embodiments, the technical solutions according to embodiments of this application may also be applied to a scenario in which the pressure bag is filled with the liquid.

As mentioned above, the electronic sphygmomanometer measures blood pressure through airbag inflation and deflation. The solenoid valve is a key accessory for controlling the connection and disconnection of the air path of the electronic sphygmomanometer. Air tightness of the solenoid valve directly affects accuracy of blood pressure measurement. In an existing electronic sphygmomanometer, to meet a sealability requirement, a size of a solenoid valve is large. With continuous improvement of living standards, people pay more attention to their own health, and require that blood pressure can be dynamically measured anytime and anywhere. The electronic sphygmomanometer is evolved to be minimized and wearable. However, a large-sized solenoid valve is difficult to meet an extreme space requirement of the electronic sphygmomanometer evolving to be minimized and wearable.

In view of this, an embodiment of this application provides a solenoid valve, to achieve miniaturization of the solenoid valve. When the solenoid valve is used in an electronic sphygmomanometer, it helps reduce a size of the electronic sphygmomanometer, and meet a user requirement.

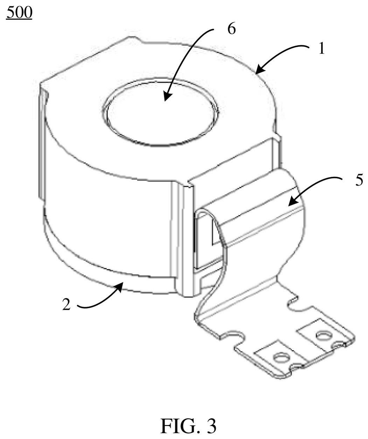

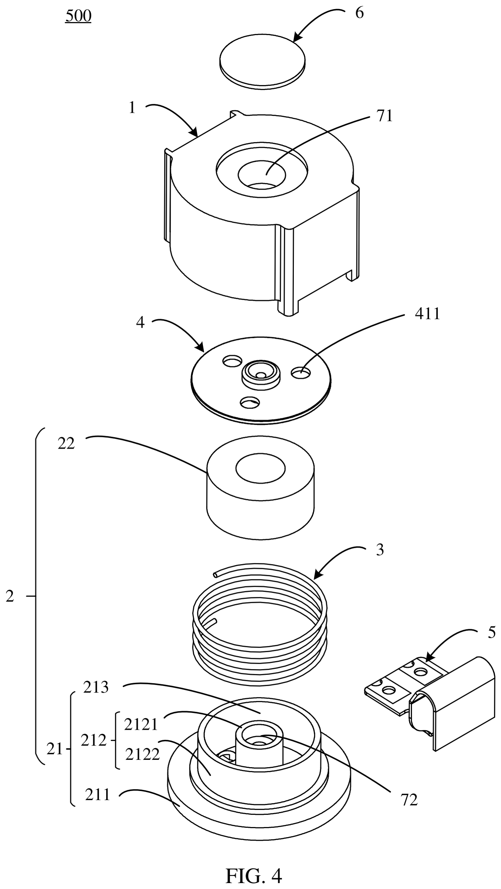

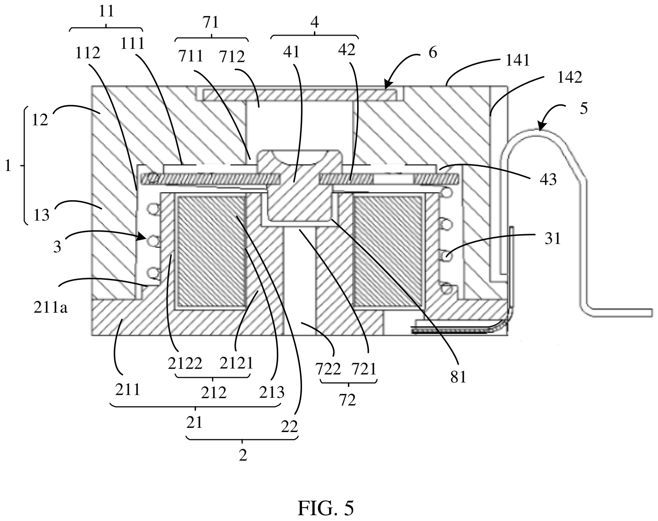

FIG. 3 to FIG. 5 are diagrams of structures of a solenoid valve according to embodiments of this application. Specifically, FIG. 3 is an assembly diagram of the solenoid valve according to an embodiment of this application, FIG. 4 is an exploded diagram of the solenoid valve according to an embodiment of this application, and FIG. 5 is a cross-sectional diagram of the solenoid valve according to an embodiment of this application.

With reference to FIG. 3 to FIG. 5, the solenoid valve 500 mainly includes a housing 1, an electromagnetic component 2, a reset component 3, and a valve plug component 4. A first medium flow channel 71 is disposed on the housing 1. The electromagnetic component 2 includes a magnetic yoke 21 and a coil 22. A second medium flow channel 72 and a space for accommodating the coil 22 are disposed on the magnetic yoke 21. The electromagnetic component 2 and the housing 1 are fastened to form an accommodation cavity. The accommodation cavity is configured to accommodate the reset component 3 and the valve plug component 4. The valve plug component 4 is disposed between the electromagnetic component 2 and the housing 1, and the reset component 3 is disposed on a side that is of the valve plug component 4 and that faces (or is close to) the electromagnetic component 2 and/or a side that is of the valve plug component 4 and that is away from the electromagnetic component 2. The electromagnetic component 2 is configured to drive the valve plug component 4 to move when the coil 22 is powered on, and the reset component 3 is configured to drive the valve plug component 4 to move when the coil 22 is powered off, to enable the first medium flow channel 71 to communicate with or be isolated from the second medium flow channel 72.

In this embodiment of this application, when the electromagnetic component 2 is fastened to the housing 1, the magnetic yoke 21 is directly fastened to the housing 1. The magnetic yoke 21 is actually used as a part of an outer housing of the solenoid valve 500. The magnetic yoke 21 is fastened to the housing 1 to form an accommodation space for accommodating another component. The component is simplified, and therefore a structure is simple. This helps reduce a size of the entire solenoid valve, to meet a space requirement of an electronic device, and achieve miniaturization of the electronic device or improve space utilization inside the electronic device. The solenoid valve according to embodiments of this application has the simple structure, so that a manufacturing process is simpler and more reliable, and dimensional precision of a component of the solenoid valve can be improved, thereby facilitating assembly of the solenoid valve and improving a yield rate. In addition, the medium flow channels are disposed on the housing 1 and the magnetic yoke 21. This facilitates overall sealing, to improve air tightness of the solenoid valve, and correspondingly improve anti-corrosion performance and reliability. Because sealability of the solenoid valve is improved, when the solenoid valve is used in an electronic sphygmomanometer, air leakage can be reduced or avoided, thereby reducing or avoiding an unstable pressure difference, and improving measurement accuracy of the electronic sphygmomanometer.

The following describes in more detail the structure of the solenoid valve according to embodiments of this application with reference to the accompanying drawings.

Refer to FIG. 4 and FIG. 5. The magnetic yoke 21 may include a first body 211 and a protrusion 212 protruding from a surface of the first body 211. Specifically, the protrusion 212 protrudes from the surface of the first body 211 towards the housing 1 (or the valve plug component 4). The first body 211 may be connected to the housing 1. A first accommodation space 213 whose opening faces the valve plug component 4 is disposed on the protrusion 212, and the first accommodation space 213 is configured to accommodate the coil 22.

Refer to FIG. 5. A first groove 11 is disposed on a side that is of the housing 1 and that faces the magnetic yoke 21, and the first groove 11 is configured to accommodate the protrusion 212 and the coil 22 accommodated in the first accommodation space 213. In other words, the housing 1 may include a second body 12 and an extension part 13 extending from a periphery (or an edge) of the second body 12 to a side of the magnetic yoke 21. The second body 12 and the extension part 13 may form the first groove 11 whose opening faces the magnetic yoke 21. In some embodiments, the first body 211 may alternatively be accommodated in the first groove 11.

When the housing 1 is connected to the magnetic yoke 21, that is, the housing 1 is fastened to the magnetic yoke 21, the coil 22 is accommodated in the first accommodation space 213 disposed on the protrusion 212, and the protrusion 212 is accommodated in the first groove 11 disposed on the housing 1. The valve plug component 4 is also accommodated in the first groove 11, and at least a part of the valve plug component 4 is located between the protrusion 212 and a bottom wall 111 of the first groove 11. Herein, the first body 211 is connected to the housing 1, the protrusion 212 is accommodated in the first groove 11, and the foregoing accommodation cavity may be formed between the housing 1 and the magnetic yoke 21 on which the coil 22 is mounted. In this embodiment of this application, the first medium flow channel 71 and the second medium flow channel 72 may be understood as a part of the accommodation cavity.

Herein, the first accommodation space 213 for accommodating the coil 22 is disposed on the magnetic yoke 21, so that a single component encloses the coil 22, and the magnetic yoke 21 does not need to cooperate with another component to form a mounting groove for accommodating the coil 22. This facilitates mounting of the coil 22, the assembly of the solenoid valve, and miniaturization of the solenoid valve, effectively saves space, facilitates overall sealing, and improves the sealability of the solenoid valve. In addition, the magnetic yoke 21 can transmit a magnetic line generated by the power-on coil to a required place, to form a magnetic field at the required place, thereby reducing magnetic leakage.

In some embodiments, the housing 1 may be fastened to the magnetic yoke 21 through welding, bonding, clamping, a thread connection, or the like. For example, the first body 211 may be fastened to the extension part 13, to connect the housing 1 to the magnetic yoke 21. In this embodiment of this application, the housing 1 is in sealing connection to the magnetic yoke 21. For example, a part for connecting the housing 1 to the magnetic yoke 21 may be sealed through adhesive dispensing, a double-sided tape, a deformed material, or the like to implement overall sealing of the solenoid valve.

In some embodiments, the housing 1 is of an integral structure (or referred to as a non-removable structure), for example, the housing 1 is integrally formed.

In some embodiments, the magnetic yoke 21 is of an integral structure (or referred to as a non-removable structure), for example, the magnetic yoke 21 is integrally formed.

The integrated housing 1 and/or magnetic yoke 21 can simplify a processing process.

In some embodiments, the first body 211 may be connected to an end face of the extension part 13 (that is, an end face that is of the extension part 13 and that is away from the second body 12 in a fastening direction). In this way, the first body 211 may be located outside the first groove 11. Alternatively, the first body 211 may be connected to a side surface (that is, a side wall 112 of the first groove 11) of the extension part 13. In this way, the first body 211 is also accommodated in the first groove 11.

Still refer to FIG. 5. The first medium flow channel 71 is disposed on the housing 1, and the first medium flow channel 71 communicates with the first groove 11. The second medium flow channel 72 is disposed on the magnetic yoke 21, and the second medium flow channel 72 communicates with the first groove 11. Therefore, the first medium flow channel 71 can communicate with the second medium flow channel 72 via the first groove 11. Whether the first medium flow channel 71 can communicate with the second medium flow channel 72 via the first groove 11 is controlled by motion of the valve plug component 4.

In this embodiment of this application, the valve plug component 4 is disposed between the electromagnetic component 2 and the housing 1, and the valve plug component 4 may move in the fastening direction of the housing 1 and the magnetic yoke 21 under action of the electromagnetic component 2 and the reset component 3, to control whether the first medium flow channel 71 communicates with the second medium flow channel 72. For example, when the valve plug component 4 blocks, under action of the electromagnetic component 2 or the reset component 3, a location at which the first medium flow channel 71 communicates with the first groove 11 or a location at which the second medium flow channel 72 communicates with the first groove 11, a path between the first medium flow channel 71 and the second medium flow channel 72 is blocked, that is, the first medium flow channel 71 does not communicate with the second medium flow channel 72. This may also be referred to as that the solenoid valve is in an open state. When the valve plug component 4 neither blocks, under the action of the electromagnetic component 2 or the reset component 3, the location at which the first medium flow channel 71 communicates with the first groove 11 nor the location at which the second medium flow channel 72 communicates with the first groove 11, the first medium flow channel 71 can communicate with the second medium flow channel 72 via the first groove 11. This may also be referred to as that the solenoid valve is in an enabled state or an open state. In other words, the valve plug component 4 is configured to move in the fastening direction of the housing 1 and the magnetic yoke 21 under the action of the electromagnetic component 2 and the reset component 3, to control the path constituted by the first medium flow channel 71 and the second medium flow channel 72 via the first groove 11 to be opened or blocked.

Herein, the first medium flow channel 71 and the second medium flow channel 72 are respectively disposed on the housing 1 and the magnetic yoke 21, and after the housing 1 is fastened to the magnetic yoke 21, a space for communicating the first medium flow channel 71 and the second medium flow channel 72 is formed. In this embodiment of this application, because the accommodation space is formed by connecting the housing 1 and the magnetic yoke 21, there are a few sealing interfaces, and sealing reliability is improved. Therefore, sealability of the entire path is good.

In some embodiments, refer to FIG. 5. The first medium flow channel 71 disposed on the housing 1 may include a first opening 711 and a second opening 712. The first opening 711 is disposed on the bottom wall 111 or the side wall 112 of the first groove 11, and the second opening 712 is disposed on a wall (that is, an outer wall of the housing 1) of the housing 1 other than the bottom wall 111 and the side wall 112 of the first groove 11. In other words, the first medium flow channel 71 communicates with the first groove 11, and the first opening 711 may be formed on the bottom wall 111 or the side wall 112 of the first groove 11. The first medium flow channel 71 communicates with the outside of the housing 1 (that is, the outside of the solenoid valve), and the second opening 712 may be formed on the outer wall of the housing 1. In this embodiment of this application, the side wall 112 and the bottom wall 111 of the first groove 11 (or walls of the housing 1 that are used to form the accommodation space when the housing 1 is fastened to the magnetic yoke 21) may be understood as inner walls of the housing 1, and a wall that is of the housing 1 and that is away from the first groove 11 (or a wall that is of the housing 1 and that is exposed to the outside when the housing 1 is fastened to the magnetic yoke 21) may be understood as the outer wall of the housing 1.

By way of example rather than limitation, for example, the first opening 711 may be disposed on the bottom wall 111 of the first groove 11, and the second opening 712 may be disposed on an outer wall 141 (which may also be referred to as a first outer wall 141 for ease of description) that is of the second body 12 and that is away from the first groove 11. For example, the first opening 711 may be disposed on the bottom wall 111 of the first groove 11, and the second opening 712 may be disposed on an outer wall 142 (which may also be referred to as a second outer wall 142 for ease of description) that is of the extension part 13 and that is away from the first groove 11. For another example, the first opening 711 may be disposed on the side wall 112 of the first groove 11, and the second opening 712 may be disposed on the first outer wall 141. For another example, the first opening 711 may be disposed on the side wall 112 of the first groove 11, and the second opening 712 may be disposed on the second outer wall 142. When the first opening 711 is disposed on the bottom wall 111 of the first groove 11, and the second opening 712 is disposed on the first outer wall 141, the first medium flow channel 71 may be configured through puncturing, and a process is simple.

It may be understood that, in this embodiment of this application, because the first medium flow channel 71 communicates the first groove 11 with the outside of the housing 1, the first opening 711 and the second opening 712 of the first medium flow channel 71 are not simultaneously disposed on the bottom wall 111 and/or the side wall 112 of the first groove 11. In other words, the first opening 711 and the second opening 712 are not simultaneously disposed on the bottom wall 111 of the first groove 11 or simultaneously disposed on the side wall 112 of the first groove 11. In addition, a case in which one opening is disposed on the bottom wall 111 of the first groove 11, and the other opening is disposed on the side wall 112 of the first groove 11 does not occur.

Herein, a single component, namely, the housing 1, forms the complete first medium flow channel 71, so that sealability of a pipe of the solenoid valve can be improved, and the assembly can be facilitated. In addition, when the housing 1 is of the integral structure, the processing process of the housing 1 is simpler.

In some embodiments, the first medium flow channel 71 may be in a form of a through hole, that is, a center line of the first medium flow channel 71 is a straight line. For example, the first medium flow channel 71 may be a cylindrical hole, a conical hole, a stepped hole, a threaded hole, or the like.

The first medium flow channel 71 in the form of the through hole has little resistance to an operating medium (such as gas or liquid) of the solenoid valve, so that the operating medium can smoothly pass through the first medium flow channel 71, thereby improving a response speed and pressure relief efficiency of the solenoid valve, and shortening pressure relief time.

In some embodiments, refer to FIG. 5. The second medium flow channel 72 disposed on the magnetic yoke 21 may include a third opening 721 and a fourth opening 722. The third opening 721 is disposed on a surface that is of the protrusion 212 and that faces the bottom wall 111 of the first groove 11, and the fourth opening 722 is disposed on a surface that is of the first body 211 and that is away from the bottom wall 111 of the first groove 11. In this way, the second medium flow channel 72 may be configured through puncturing, and a processing process is simple. It may be understood that, the third opening 721 is different from an opening of the first accommodation space 213, that is, the third opening 721 and the opening of the first accommodation space 213 are disposed at different locations on the surface that is of the protrusion 212 and that faces the bottom wall 111 of the first groove 11.

Herein, a single component, namely, the magnetic yoke 21, forms the complete second medium flow channel 72, so that sealability of a pipe of the solenoid valve can be improved, and the assembly can be facilitated. In addition, when the magnetic yoke 21 is of the integral structure, the processing process of the magnetic yoke 21 is simpler.

In some embodiments, the second medium flow channel 72 may be in a form of a through hole, that is, a center line of the second medium flow channel 72 is a straight line. For example, the second medium flow channel 72 may be a cylindrical hole, a conical hole, a stepped hole, a threaded hole, or the like.

The second medium flow channel 72 in the form of the through hole has little resistance to the operating medium (such as the gas or the liquid) of the solenoid valve, so that the operating medium can smoothly pass through the second medium flow channel 72, thereby improving the response speed and the pressure relief efficiency of the solenoid valve, and shortening the pressure relief time.

In some embodiments, the second medium flow channel 72 and the first medium flow channel 71 are disposed coaxially. Alternatively, in other words, the center line of the second medium flow channel 72 overlaps the center line of the first medium flow channel 71. In this way, during pressure relief, the operating medium of the solenoid valve can smoothly reach an outlet from an inlet of the solenoid valve, a flow distance is short, the resistance is low, the response speed and the pressure relief efficiency of the solenoid valve are improved, and the pressure relief time is shortened.

In some embodiments, at least one first accommodation space 213 may be disposed on the protrusion 212, the electromagnetic component 2 may include at least one coil 22, and the at least one first accommodation space 213 one-to-one corresponds to the at least one coil 22. Specifically, one of the at least one first accommodation space 213 is configured to accommodate one of the at least one coil 22.

For example, a plurality of first accommodation spaces 213 may be disposed on the protrusion 212, and the plurality of first accommodation spaces 213 may be arranged in an annular array (for example, a circular ring, a square ring, or a triangular ring). Correspondingly, coils 22 accommodated in the plurality of first accommodation spaces 213 may form a coil array. When a plurality of coils 22 are disposed in the solenoid valve, the plurality of coils 22 may be divided into a plurality of groups to operate in turn. When one group of coils is faulty, another group of coils may continue to operate, which helps prolong a service life of the solenoid valve.

For another example, one first accommodation space 213 may be disposed on the protrusion 212. Refer to FIG. 4 and FIG. 5. The protrusion 212 may include a first protrusion 2121 and a second protrusion 2122, the second protrusion 2122 is disposed around the first protrusion 2121, a groove is formed between the first protrusion 2121 and the second protrusion 2122, and the groove is the first accommodation space 213 configured to accommodate the coil 22. For example, the first accommodation space 213 may be a ring (for example, a circular ring, a square ring, or a triangular ring), and the coil 22 accommodated in the first accommodation space 213 may be a ring coil. When a single coil 22 is disposed in the solenoid valve, a circuit connection and a cabling layout can be simplified.

In some embodiments, the third opening 721 of the second medium flow channel 72 may be disposed on a surface that is of the first protrusion 2121 and that faces the bottom wall 111 of the first groove 11. In this way, when the valve plug component 4 is configured to block or open the third opening 721, the valve plug component 4 is evenly supported by a force, so that blocking effect of the valve plug component 4 can be improved.

In some embodiments, the groove (that is, the first accommodation space 213) between the first protrusion 2121 and the second protrusion 2122 is disposed around the second medium flow channel 72. Correspondingly, the coil 22 accommodated in the groove is disposed around the second medium flow channel 72.

The reset component 3 may be disposed in a plurality of manners. As described above, the reset component 3 may be disposed on the side that is of the valve plug component 4 and that faces the electromagnetic component 2 and/or the side that is of the valve plug component 4 and that is away from the electromagnetic component 2.

For example, the reset component 3 may be disposed on the side that is of the valve plug component 4 and that faces the electromagnetic component 2, that is, the reset component 3 and the electromagnetic component 2 may be disposed on the same side of the valve plug component 4.

For example, refer to FIG. 4 and FIG. 5. The reset component 3 may include an elastic member 31, and the elastic member 31 is disposed on a periphery of the protrusion 212. For example, the elastic member 31 is sleeved on the protrusion 212. It may be understood that, when the protrusion 212 includes the first protrusion 2121 and the second protrusion 2122, the elastic member 31 is sleeved on the second protrusion 2122. One end of the elastic member 31 presses against the valve plug component 4, and the other end of the elastic member 31 presses against the first body 211. More specifically, one end of the elastic member 31 presses against a surface that is of the valve plug component 4 and that faces the coil 22 (or the elastic member 31), and the other end of the elastic member 31 presses against a surface that is of the first body 211 and that faces the valve plug component 4.

In some embodiments, a surface that is of the first body 211 and that faces the valve plug component 4 may be a plane, and the elastic member 31 may press against the plane. In some other embodiments, as shown in FIG. 5, the surface that is of the first body 211 and that faces the valve plug component 4 may be a stepped surface, that is, a step is disposed between the first body 211 and the protrusion 212, and the elastic member 31 may press against the stepped surface. In this way, when a connection part between the first body 211 and the extension part 13 is sealed through adhesive dispensing, the adhesive dispensing can be prevented from overflowing to the elastic member 31, and affecting performance of the elastic member 31.

In this embodiment, when the coil 22 is not powered on (that is, the coil 22 does not operate), the elastic member 31 is in a compressed state. Under action of an elastic force of the elastic member 31, the valve plug component 4 abuts against the bottom wall 111 of the first groove 11. After the coil 22 is powered on (that is, the coil 22 operates), the coil 22 generates a magnetic field. The magnetic field may magnetize (or be referred to as polarize) the magnetic yoke 21, so that the magnetic yoke 21 generates a magnetic force. The valve plug component 4 includes a magnetic material. The magnetic material can be attracted by the magnetized magnetic yoke 21. A magnetic attraction force of the magnetic yoke 21 is greater than the elastic force of the elastic member 31. Therefore, the magnetized magnetic yoke 21 can overcome the elastic force of the elastic member 31 to attract the valve plug component 4, to drive the valve plug component 4 to move towards a side of the coil 22, and the elastic member 31 is further compressed. After the coil 22 is powered off (that is, the coil 22 does not operate), the magnetic field of the coil 22 disappears, the magnetic force of the magnetic yoke 21 disappears, the magnetic attraction force between the magnetic yoke 21 and the valve plug component 4 disappears, and the elastic member 31 tends to restore. Therefore, under the action of the elastic force of the elastic member 31, the valve plug component 4 moves towards a side of the bottom wall 111 of the first groove 11, that is, moves in a direction away from the coil 22, and finally abuts against the bottom wall 111 of the first groove 11.

In some embodiments, when the coil 22 is powered on, the magnetized magnetic yoke 21 drives the valve plug component 4 to block the second medium flow channel 72, to block the path between the first medium flow channel 71 and the second medium flow channel 72. When the coil 22 is not powered on, the elastic member 31 drives the valve plug component 4 to open the second medium flow channel 72, to communicate the first medium flow channel 71 with the second medium flow channel 72.

For ease of understanding, with reference to FIG. 5, the following describes an operating process of the solenoid valve by using an example in which the second medium flow channel 72 communicates with an airbag. When the electronic sphygmomanometer does not operate, the coil 22 is not powered on. Under the action of the elastic force of the elastic member 31, the valve plug component 4 abuts against the bottom wall 111 of the first groove 11, and the valve plug component 4 neither blocks the third opening 721 of the second medium flow channel 72 nor the first opening 711 of the first medium flow channel 71. Therefore, the first medium flow channel 71 communicates with the second medium flow channel 72 via the first groove 11, and the solenoid valve is in the open state. When the electronic sphygmomanometer performs inflation for the entire device, for example, a user taps a measurement button on a display of the electronic sphygmomanometer or presses a measurement key of the electronic sphygmomanometer, an air pump inflates the airbag, and the coil 22 is powered on. Under the action of the magnetic force of the magnetic yoke 21, the valve plug component 4 moves towards the side of the coil 22, and finally abuts against the protrusion 212. The valve plug component 4 blocks the third opening 721 of the second medium flow channel 72. Therefore, the first medium flow channel 71 does not communicate with the second medium flow channel 72, the solenoid valve is in the closed state, and gas in the airbag cannot be discharged via the solenoid valve. When the electronic sphygmomanometer performs deflation for the entire device, the coil 22 is powered off, and the magnetic force of the magnetic yoke 21 disappears. Under the action of the elastic force of the elastic member 31, the valve plug component 4 moves towards a side away from the coil 22, and finally abuts against the bottom wall 111 of the first groove 11. In this case, the first medium flow channel 71 communicates with the second medium flow channel 72 via the first groove 11, the solenoid valve is in the open state, and the gas in the airbag can be discharged via the solenoid valve. The second medium flow channel 72 is a gas inlet of the solenoid valve, and the first medium flow channel 71 is a gas outlet of the solenoid valve.

It may be understood that, in some other embodiments, the first medium flow channel 71 may alternatively communicate with the airbag, and an operating process of the solenoid valve is the same as that described above. However, a flow direction of the gas is different when the airbag is deflated. For brevity, details are not described herein again.

In this example, after the coil 22 is powered on, the solenoid valve is in the closed state, so that sealability of a pipeline in which the solenoid valve is located can be ensured, thereby maintaining pressure of the pipeline in which the solenoid valve is located. After the coil 22 is powered off, the solenoid valve is in the open state, and the pressure of the pipeline in which the solenoid valve is located may be discharged. When the coil 22 is always not powered on, the solenoid valve is in a normally open state.

In some other embodiments, when the coil 22 is not powered on, the elastic member 31 drives the valve plug component 4 to block the first medium flow channel 71, to block the path between the first medium flow channel 71 and the second medium flow channel 72. When the coil 22 is powered on, the magnetized magnetic yoke 21 drives the valve plug component 4 to open the first medium flow channel 71, to communicate the first medium flow channel 71 with the second medium flow channel 72.

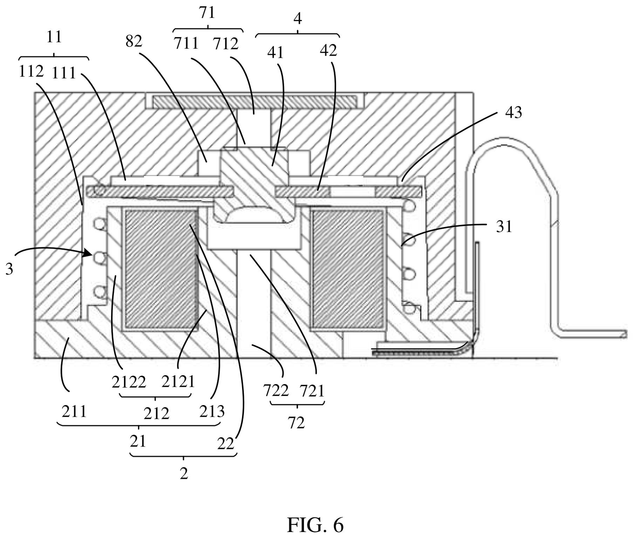

For ease of understanding, with reference to FIG. 6, the following describes an operating process of the solenoid valve by using an example in which the first medium flow channel 71 communicates with an airbag. When the electronic sphygmomanometer does not operate, the coil 22 is not powered on. Under the action of the elastic force of the elastic member 31, the valve plug component 4 abuts against the bottom wall 111 of the first groove 11, and the valve plug component 4 blocks the first opening 711 of the first medium flow channel 71. Therefore, the first medium flow channel 71 does not communicate with the second medium flow channel 72, and the solenoid valve is in the open state. When the electronic sphygmomanometer performs inflation for the entire device, for example, a user taps a measurement button on a display of the electronic sphygmomanometer or presses a measurement key of the electronic sphygmomanometer, an air pump inflates the airbag, and the coil 22 maintains a power-off state. The solenoid valve is in the open state, and gas in the airbag cannot be discharged via the solenoid valve. When the electronic sphygmomanometer performs deflation for the entire device, the coil 22 is powered on. Under the action of the magnetic force of the magnetic yoke 21, the valve plug component 4 moves towards the side of the coil 22, and finally abuts against the protrusion 212. The valve plug component 4 neither blocks the first opening 711 of the first medium flow channel 71 nor the third opening 721 of the second medium flow channel 72. Therefore, the first medium flow channel 71 communicates with the second medium flow channel 72 via the first groove 11, the solenoid valve is in the closed state, and the gas in the airbag may be discharged via the solenoid valve. The first medium flow channel 71 is a gas inlet of the solenoid valve, and the second medium flow channel 72 is a gas outlet of the solenoid valve.

It may be understood that, in some other embodiments, the second medium flow channel 72 may alternatively communicate with the airbag, and an operating process of the solenoid valve is the same as that described above. However, a flow direction of the gas is different when the airbag is deflated. For brevity, details are not described herein again.

In this example, after the coil 22 is powered on, the solenoid valve is in the closed state, and pressure of a pipeline in which the solenoid valve is located may be discharged. After the coil 22 is powered off, the solenoid valve is in the open state, so that sealability of the pipeline in which the solenoid valve is located can be ensured, thereby maintaining the pressure of the pipeline in which the solenoid valve is located. When the coil 22 is always not powered on, the solenoid valve is in a normally open state.

In some embodiments, the elastic member 31 may be a spring, a corrugated pipe, an elastic block, another element made of an elastic material (for example, spring steel, rubber, or latex), or the like. When the elastic member 31 is the spring, the elastic member 31 may be specifically a metal spring, for example, a cylindrical helical spring, a conical helical spring, a barrel shaped helical spring, an hourglass shaped helical spring, a wave spring, or a disc spring, or may be a non-metal spring, for example, an air spring or a rubber spring.

A spring diameter and a spring pitch of the cylindrical helical spring are not changed, and a spring feature is linear. The conical helical spring, the barrel shaped helical spring, and the hourglass shaped helical spring are all variable-diameter helical springs with good cushioning performance. The wave spring (WP for short) is an elastic element with several peaks and valleys on a thin metal ring. The disc spring, also known as a Bellville spring washer, is shaped as a conical disc, which can be used independently. Alternatively, a plurality of disc springs can be used in series or in parallel. The upper inner edge and lower outer edge bear static or dynamic load in an axial direction. The air spring is a spring that achieves, based on compressibility of air, elastic effect by filling compressed air in a telescopic airtight container. The rubber spring is a type of polymer elastomer. The rubber spring is made of common rubber, which has large elastic deformation and a strong restoration capability. The rubber spring can absorb vibration and shock generated by a machine, noise generated by operation vibration, and the like. The corrugated pipe is a tubular elastic element formed by connecting foldable corrugated sheets in a folding and telescopic direction.

The elastic member 31 has a good elastic deformation capability, and is easy to process and arrange. When the coil 22 is not powered on, the elastic member 31 is used to drive the valve plug component 4 to move and maintain a stable state of the valve plug component 4, so that good reliability is provided.

In some embodiments, refer to FIG. 5 or FIG. 6. The valve plug component 4 may include a sealing member 41 and a separator 42. The sealing member 41 is configured to block the first medium flow channel 71 or the second medium flow channel 72, and the separator 42 is configured to drive, under driving of the electromagnetic component 2 or the reset component 3, the sealing member 41 to move, to block the first medium flow channel 71 or the second medium flow channel 72.

For example, the structure shown in FIG. 5 is used as an example, the reset component 3 includes the elastic member 31. When the coil 22 is powered on, the coil 22 generates the magnetic field, and the magnetic field polarizes the magnetic yoke 21, so that the magnetic yoke 21 generates the magnetic force. In this case, the magnetic force of the magnetic yoke 21 is greater than the elastic force of the elastic member 31, so that the magnetic yoke 21 can attract the separator 42 to move towards the side of the coil 22. Correspondingly, the separator 42 drives the sealing member 41 to move towards the side of the coil 22. After the separator 42 reaches a first stable state, for example, the separator 42 abuts against the protrusion 212, a location of the sealing member 41 may be fixed, that is, motion of the sealing member 41 is limited, and the sealing member 41 maintains a state of blocking the second medium flow channel 72, that is, the sealing member 41 maintains a state in which the flow channel (or pipeline) of the solenoid valve is blocked. When the coil 22 is powered off, the magnetic field of the coil 22 disappears, the magnetic force of the magnetic yoke 21 disappears, and the separator 42 moves in the direction away from the coil 22 under the action of the elastic force of the elastic member 31. Correspondingly, the separator 42 drives the sealing member 41 to move in the direction away from the coil 22. After the separator 42 reaches a second stable state, for example, the separator 42 abuts against the bottom wall 111 of the first groove 11, the location of the sealing member 41 may be fixed, that is, the motion of the sealing member 41 is limited. The sealing member 41 neither blocks the second medium flow channel 72 nor the first medium flow channel 71, that is, the sealing member 41 maintains a state in which the flow channel (or pipeline) of the solenoid valve is opened.

For another example, the structure shown in FIG. 6 is used as an example, the reset component 3 includes the elastic member 31. When the coil 22 is powered on, the coil 22 generates the magnetic field, and the magnetic field polarizes the magnetic yoke 21, so that the magnetic yoke 21 generates the magnetic force. In this case, the magnetic force of the magnetic yoke 21 is greater than the elastic force of the elastic member 31, so that the magnetic yoke 21 can attract the separator 42 to move towards the side of the coil 22. Correspondingly, the separator 42 drives the sealing member 41 to move towards the side of the coil 22. After the separator 42 reaches a second stable state, for example, the separator 42 abuts against the protrusion 212, a location of the sealing member 41 may be fixed. The sealing member 41 neither blocks the second medium flow channel 72 nor the first medium flow channel 71, that is, the sealing member 41 maintains a state in which the flow channel (or pipeline) of the solenoid valve is opened. When the coil 22 is powered off, the magnetic field of the coil 22 disappears, the magnetic force of the magnetic yoke 21 disappears, and the separator 42 moves in the direction away from the coil 22 under the action of the elastic force of the elastic member 31. Correspondingly, the separator 42 drives the sealing member 41 to move in the direction away from the coil 22. After the separator 42 reaches a first stable state, for example, the separator 42 abuts against the bottom wall 111 of the first groove 11, the location of the sealing member 41 may be fixed. The sealing member 41 maintains a state in which the first medium flow channel 71 is blocked, that is, the sealing member 41 maintains a state in which the flow channel (or pipeline) of the solenoid valve is blocked.

In some embodiments, the sealing member 41 fastened to the separator 42. For example, the separator 42 may be fastened to the sealing member 41 through welding, clamping, bonding, a thread connection, a key connection, a pin connection, an interference fit, or the like. By way of example rather than limitation, the separator 42 may be sleeved on the sealing member 41, and is fastened to the sealing member 41 in at least one of the foregoing manners. In this case, the sealing member 41 and the separator 42 may be prepared based on different materials for respective functions.

In some other embodiments, the sealing member 41 and the separator 42 may be integrally formed. In this case, an assembly process of the valve plug component 4 may be simplified, and the assembly is facilitated.

In some embodiments, the sealing member 41 may be made of a metal material (such as aluminum, lead, indium, or stainless steel), a non-metal material (such as rubber, silicone, plastics, ceramic, graphite, or synthetic resin), or a composite material (such as a rubber-asbestos gasket or aerogel felt-polyurethane). For example, the sealing member 41 may be made of an elastic material (such as rubber or a thermoplastic elastomer). In this way, the sealing member 41 has given elasticity. When the first medium flow channel 71 or the second medium flow channel 72 is blocked, the sealing member 41 is deformed to some extent, and has good sealability.

In some embodiments, if the sealing member 41 is configured to block the second medium flow channel 72, an end that is of the sealing member 41 and that is close to the second medium flow channel 72 (or close to the third opening 721) may protrude in a direction that is relative to the separator 42 and that is towards the third opening 721. This helps the sealing member 41 form good sealing at the third opening 721.

For example, refer to FIG. 5. A second groove 81 may be disposed on a side that is of the protrusion 212 (for example, the first protrusion 2121) and that faces the bottom wall 111 of the first groove 11, and the second medium flow channel 72 communicates with the second groove 81 and forms the third opening 721 on a bottom wall of the second groove 81. In other words, the second medium flow channel 72 may communicate with the first groove 11 via the second groove 81. The second groove 81 is configured to accommodate a part, of the sealing member 41, protruding in the direction that is relative to the separator 42 and that is towards the third opening 721. In this way, thickness of the solenoid valve in a direction in which the housing 1 is fastened to the magnetic yoke 21 can be reduced while the sealability of the sealing member 41 is met, thereby helping achieve the miniaturization of the solenoid valve. In addition, the sealing member 41 has a large size in the fastening direction, and can have a large deformation amount, so that good blocking effect can be achieved. In addition, during blocking, a small current is applied to the coil 22 to achieve the good blocking effect, and power consumption of the solenoid valve can be reduced.

It may be understood that, in this case, a radial size (for example, a diameter) of the part, of the sealing member 41, protruding in the direction that is relative to the separator 42 and that is towards the third opening 721 is greater than a radial size (for example, a diameter) of the third opening 721. In this way, the sealing member 41 can block the third opening 721. In addition, there is a given gap between a side wall of the second groove 81 and the part, of the sealing member 41, protruding in the direction that is relative to the separator 42 and that is towards the third opening 721, to avoid friction, from affecting motion of the separator 42, generated between the side wall of the second groove 81 and the part, of the sealing member 41, protruding in the direction that is relative to the separator 42 and that is towards the third opening 721.

In some embodiments, if the sealing member 41 is configured to block the first medium flow channel 71, an end that is of the sealing member 41 and that is close to the first medium flow channel 71 (or close to the first opening 711) may protrude in a direction that is relative to the separator 42 and that is towards the first opening 711. This helps the sealing member 41 form good sealing at the first opening 711.

For example, refer to FIG. 6. A third groove 82 may be disposed on the side that is of the housing 1 and that faces the magnetic yoke 21, and an opening of the third groove 82 is located on the bottom wall 111 of the first groove 11. In other words, the third groove 82 and the first groove 11 may form a stepped groove. Alternatively, the third groove 82 is disposed on the bottom wall 111 of the first groove 11. The first medium flow channel 71 communicates with the third groove 82, and the first opening 711 is formed on the bottom wall of the third groove 82. In other words, the first medium flow channel 71 may communicate with the first groove 11 via the third groove 82. The third groove 82 is configured to accommodate a part, of the sealing member 41, protruding in the direction that is relative to the separator 42 and that is towards the first opening 711. In this way, thickness of the solenoid valve in a direction in which the housing 1 is fastened to the magnetic yoke 21 can be reduced while the sealability of the sealing member 41 is met, thereby helping achieve the miniaturization of the solenoid valve.

It may be understood that, in this case, a radial size (for example, a diameter) of the part, of the sealing member 41, protruding in the direction that is relative to the separator 42 and that is towards the first opening 711 is greater than a radial size (for example, a diameter) of the first opening 711. In this way, the sealing member 41 can block the first opening 711. In addition, there is a given gap between a side wall of the third groove 82 and the part, of the sealing member 41, protruding in the direction that is relative to the separator 42 and that is towards the first opening 711, to avoid friction, from affecting motion of the separator 42, generated between the side wall of the third groove 82 and the part, of the sealing member 41, protruding in the direction that is relative to the separator 42 and that is towards the first opening 711.

In some embodiments, an end that is of the sealing member 41 and that is close to an unsealed medium flow channel may also protrude in a direction that is relative to the separator 42 and that is towards an opening of the unsealed medium flow channel.

For example, refer to FIG. 5. If the sealing member 41 is configured to block the second medium flow channel 72, an end that is of the sealing member 41 and that is close to the first medium flow channel 71 (or close to the first opening 711) may protrude in a direction that is relative to the separator 42 and that is towards the first opening 711. A radial size of the first opening 711 should be greater than a radial size of a part, of the sealing member 41, protruding in the direction that is relative to the separator 42 and that is towards the first opening 711. In this way, when the separator 42 drives the sealing member 41 to move towards the direction of the first opening 711 and reaches a stable state, the sealing member 41 does not block the first opening 721, to maintain that the first medium flow channel 71 communicates with the second medium flow channel 72.

Similarly, when the sealing member 41 is configured to block the first medium flow channel 71, an end that is of the sealing member 41 and that is close to the second medium flow channel 72 (or close to the third opening 721) may protrude in a direction that is relative to the separator 42 and that is towards the third opening 721. A radial size of the third opening 721 should be greater than a radial size of a part, of the sealing member 41, protruding in the direction that is relative to the separator 42 and that is towards the third opening 721.

For another example, refer to FIG. 6. If the sealing member 41 is configured to block the first medium flow channel 71, an end that is of the sealing member 41 and that is close to the second medium flow channel 72 (or close to the third opening 721) may protrude in a direction that is relative to the separator 42 and that is towards the third opening 721. When the separator 42 abuts against the protrusion 212 and reaches a stable state, a distance between the third opening 721 and a part, of the sealing member 41, protruding in the direction that is relative to the separator 42 and that is towards the third opening 721 should be greater than 0. In this way, when the separator 42 drives the sealing member 41 to move towards the direction of the third opening 721 and reaches the stable state, the sealing member 41 does not block the third opening 721, to maintain that the first medium flow channel 71 communicates with the second medium flow channel 72.

Similarly, when the sealing member 41 is configured to block the second medium flow channel 72, an end that is of the sealing member 41 and that is close to the first medium flow channel 71 (or close to the first opening 711) may protrude in a direction that is relative to the separator 42 and that is towards the first opening 711. When the separator 42 abuts against the bottom wall 111 of the first groove 11 and reaches the stable state, a distance between the first opening 711 and a part, of the sealing member 41, protruding in the direction that is relative to the separator 42 and that is towards the first opening 711 should be greater than 0.

In some embodiments, if the sealing member 41 is configured to block the first medium flow channel 71, when the valve plug component 4 abuts against the protrusion 212, a part that is of the sealing member 41 and that protrudes towards the second medium flow channel 72 relative to the separator 42 may be at least partially accommodated in the second medium flow channel 72, and there is a gap between an inner wall of the second medium flow channel 72 and the sealing member 41. In this way, the sealing member 41 does not block the opening of the second medium flow channel 72.

In some embodiments, if the sealing member 41 is configured to block the second medium flow channel 72, when the valve plug component 4 abuts against the bottom wall 111 of the first groove 11, a part that is of the sealing member 41 and that protrudes towards the first medium flow channel 71 relative to the separator 42 may be at least partially accommodated in the first medium flow channel 71, and there is a gap between an inner wall of the first medium flow channel 71 and the sealing member 41. In this way, the sealing member 41 does not block the opening of the first medium flow channel 71.

In some embodiments, the separator 42 may include a magnetic material such as a permanent magnet material or a soft magnetic material. In this way, after the coil 22 is powered on, the separator 42 may be attracted by the magnetized magnetic yoke 21 to move.

In some embodiments, the separator 42 is in a flat shape. The separator 42 occupies a small space, and the thickness of the solenoid valve in the direction in which the housing 1 is fastened to the magnetic yoke 21 can be reduced, thereby helping achieve the miniaturization of the solenoid valve. In addition, the flat-shaped separator 42 can ensure a closed-loop magnetic circuit, thereby ensuring that the magnetic circuit is smooth.

In some embodiments, refer to FIG. 4. At least one through hole 411 may be disposed on the separator 42, and the through hole 411 is configured to communicate the first medium flow channel 71 with the second medium flow channel 72 during the pressure relief. For example, the first opening 711 of the first medium flow channel 71 and the third opening 721 of the second medium flow channel 72 are respectively located on two sides of the separator 42. In other words, when the first opening 711 of the first medium flow channel 71 is disposed on the bottom wall 111 of the first groove 11, the through hole 411 may be disposed on the separator 42 to connect the path between the housing 1 and the magnetic yoke 21.

In some embodiments, refer to FIG. 5 or FIG. 6. The protruding part 43 may be disposed on the bottom wall 111 of the first groove 11. When the coil 22 is not powered on or is powered off, the separator 42 may abut against the protruding part 43 under action of the reset component 3. In this way, there is a given gap between the separator 42 and a part that is on the bottom wall 111 of the first groove 11 and on which the protruding part 43 is not disposed, so that enough space can be provided for the operating medium to flow when the solenoid valve is in the open state. This helps to perform quick pressure relief. In addition, the separator 42 presses against the bottom wall 111 of the first groove 11 via the protruding part 43, to avoid parallelism caused by large-area support.

It may be understood that, when the separator 42 abuts against the protruding part 43, the protruding part 43 and the through hole 411 are disposed in a staggered manner. For example, projection of the protruding part 43 in the fastening direction does not overlap projection of the through hole 411 in the fastening direction, to prevent the protruding part 43 from blocking the through hole 411.

In some embodiments, the protruding part 43 may alternatively be disposed on a surface that is of the separator 42 and that faces the bottom wall 111 of the first groove 11, and the separator 42 abuts against the bottom wall 111 of the first groove 11 via the protruding part 43. In this way, a space for the operating medium to flow may alternatively be reserved between the separator 42 and the bottom wall 111 of the first groove 11.

In some embodiments, the protruding part 43 disposed on the separator 42 or the bottom wall 111 of the first groove 11 may be an integrated annular component, or may include a plurality of subparts disposed in a distributed manner (for example, disposed in an annular array). In this way, the separator 42 is more evenly supported by a force.

Alternatively, in some other embodiments, the protruding part 43 may not be disposed on the bottom wall 111 of the first groove 11 or the separator 42, and a connection channel communicating the through hole 411 with the first medium flow channel 71 is disposed on the housing 1. In this way, when the separator 42 abuts against the bottom wall 111 of the first groove 11, the operating medium may reach the first medium flow channel 71 via the through hole 411 and the connection channel disposed on the housing 1.

In some embodiments, the through hole 411 may not be disposed on the separator 42. For example, the first opening 711 of the first medium flow channel 71 and the third opening 721 of the second medium flow channel 72 are located on the same side of the separator 42. To be specific, when the first opening 711 of the first medium flow channel 71 is disposed on the side wall 112 of the first groove 11, during the pressure relief, the operating medium may flow, without passing through the separator 42, from the inlet to the outlet via a gap between the valve plug component 4 and the protrusion 212, and a gap between the side wall 112 of the first groove 11 and the protrusion 212.

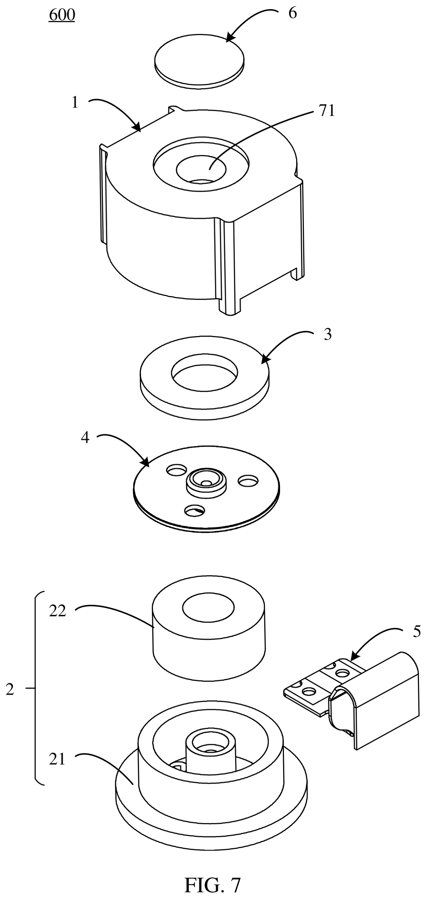

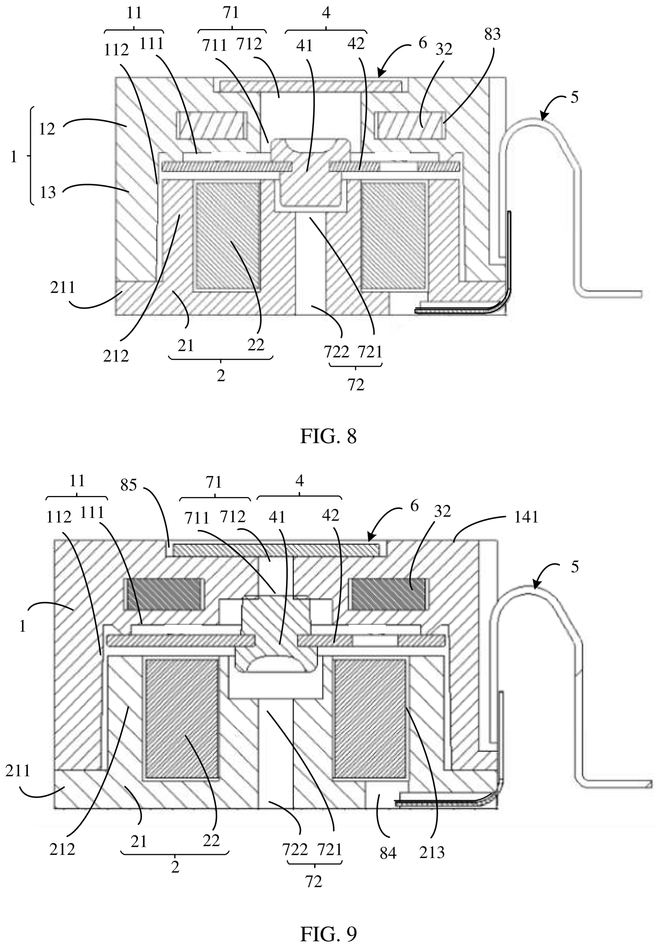

The foregoing describes a disposing manner of the reset component 3 with reference to FIG. 4 to FIG. 6. The following describes another disposing manner of the reset component 3 with reference to FIG. 7 to FIG. 9. FIG. 7 is an exploded diagram of a solenoid valve according to an embodiment of this application, and FIG. 8 and FIG. 9 are cross-sectional diagrams of a solenoid valve according to embodiments of this application. The following mainly describes differences between a solenoid valve 600 in embodiments shown in FIG. 7 to FIG. 9 and the solenoid valve 500 in embodiments shown in FIG. 4 to FIG. 6. For other components or parts that are not described in detail, refer to descriptions of corresponding parts in embodiments shown in FIG. 4 to FIG. 6.

For another example, the reset component 3 may be disposed on the side that is of the valve plug component 4 and that is away from the electromagnetic component 2, that is, the reset component 3 and the electromagnetic component 2 may be disposed on different sides of the valve plug component 4.

Refer to FIG. 7 to FIG. 9. The reset component 3 may include a magnetic member 32, and the magnetic member 32 is disposed in the housing 1. For example, a second accommodation space 83 that accommodates the magnetic member 32 may be disposed in the housing 1, and the second accommodation space 83 may be close to the bottom wall 111 of the first groove and corresponds to a location of the valve plug component 4. By way of example rather than limitation, the housing 1 may include the second body 12 and the extension part 13 extending from the periphery (or the edge) of the second body 12 to the side of the magnetic yoke 21. The second body 12 and the extension part 13 may form the first groove 11 whose opening faces the magnetic yoke 21. The second accommodation space 83 configured to accommodate the magnetic member 32 is disposed on the second body 12. For descriptions of the magnetic yoke 21, the coil 22, the first medium flow channel 71, the second medium flow channel 72, and the first groove 11, refer to the foregoing descriptions of corresponding parts. For brevity, details are not described herein again.

In some embodiments, the magnetic member 32 may be injected into the housing 1 by using an insert molding process. Injection molding is a process in which an insertion is fastened at a proper location in an injection mold in advance, and plastics are injected for molding. After the mold is opened, the insertion is tightly wrapped by the cooled and cured plastics and buried in the plastics, to obtain a product with the insertion. An integrated product made in this way avoids an addition of a new sealing interface. This helps improve sealability of the solenoid valve, and does not affect performance of the magnetic member 32.

Certainly, in some other embodiments, the second body 12 may include a first part and a second part, and the first part may be fastened to the second part to form the second accommodation space 83 configured to accommodate the magnetic member 32.

The valve plug component 4 includes the magnetic material. In this embodiment, when the coil 22 is not powered on (that is, the coil 22 does not operate), the valve plug component 4 is attracted by the magnetic member 32 to abut against the bottom wall 111 of the first groove 11 under action of a magnetic force of the magnetic member 32. When the coil 22 is powered on (that is, the coil 22 operates), the magnetic material of the valve plug component 4 can be attracted by the magnetized magnetic yoke 21. The magnetic attraction force of the magnetic yoke 21 is greater than a magnetic attraction force of the magnetic member 32. Therefore, the magnetized magnetic yoke 21 can overcome the magnetic attraction force of the magnetic member 32 to attract the valve plug component 4, to drive the valve plug component 4 to move towards the side of the coil 22. After the coil 22 is powered off (that is, the coil 22 does not operate), the magnetic field of the coil 22 disappears. Under the action of the magnetic attraction force of the magnetic member 32, the valve plug component 4 moves towards the side of the bottom wall 11 of the first groove 11, that is, moves in the direction away from the coil 22, and finally abuts against the bottom wall 111 of the first groove 11.

In some embodiments, refer to FIG. 8. When the coil 22 is powered on, the magnetized magnetic yoke 21 can overcome the magnetic attraction force between the magnetic member 32 and the valve plug component 4 to drive the valve plug component 4 to block the second medium flow channel 72, to block the path between the first medium flow channel 71 and the second medium flow channel 72. When the coil 22 is not powered on, the magnetic attraction force between the magnetic member 32 and the valve plug component 4 can drive the valve plug component 4 to open the second medium flow channel 72, to communicate the first medium flow channel 71 with the second medium flow channel 72.

An operating process of the solenoid valve shown in FIG. 8 is similar to the operating process of the solenoid valve shown in FIG. 5, and a difference lies in that: in the structure of the solenoid valve shown in FIG. 5, the valve plug component 4 moves or maintains the stable state under the action of the elastic force of the elastic member 31 and the magnetic attraction force generated between the magnetic yoke 21 and the valve plug component 4 (specifically, the separator 42 of the valve plug component 4) after the coil 22 is powered on; and in a structure of the solenoid valve shown in FIG. 8, the valve plug component 4 moves or maintains the stable state under the action of the magnetic attraction force between the magnetic member 32 and the valve plug component 4 (specifically, the separator 42 of the valve plug component 4) and the magnetic attraction force generated between the magnetic yoke 21 and the valve plug component 4 after the coil 22 is powered on. For the operating process of the solenoid valve shown in FIG. 8, refer to descriptions of related parts in FIG. 5. For brevity, details are not described herein again.

In some other embodiments, refer to FIG. 9. When the coil 22 is not powered on, the magnetic attraction force between the magnetic member 32 and the valve plug component 4 can drive the valve plug component 4 to block the first medium flow channel 71, to block the path between the first medium flow channel 71 and the second medium flow channel 72. When the coil 22 is powered on, the magnetized magnetic yoke 21 can overcome the magnetic attraction force between the magnetic member 32 and the valve plug component 4 to drive the valve plug component 4 to open the first medium flow channel 71, to communicate the first medium flow channel 71 with the second medium flow channel 72.

An operating process of the solenoid valve shown in FIG. 9 is similar to the operating process of the solenoid valve shown in FIG. 6, and a difference lies in that: in the structure of the solenoid valve shown in FIG. 6, the valve plug component 4 moves or maintains the stable state under the action of the elastic force of the elastic member 31 and the magnetic attraction force generated between the magnetic yoke 21 and the valve plug component 4 (specifically, the separator 42 of the valve plug component 4) after the coil 22 is powered on; and in a structure of the solenoid valve shown in FIG. 9, the valve plug component 4 moves or maintains the stable state under the action of the magnetic attraction force between the magnetic member 32 and the valve plug component 4 (specifically, the separator 42 of the valve plug component 4) and the magnetic attraction force generated between the magnetic yoke 21 and the valve plug component 4 after the coil 22 is powered on. For the operating process of the solenoid valve shown in FIG. 9, refer to descriptions of related parts in FIG. 6. For brevity, details are not described herein again.

In this embodiment of this application, when the reset component 3 uses the magnetic member 32, the valve plug component 4 moves under the action of the magnetic force. In this way, the valve plug component 4 can be more evenly supported by the force and move more stably. In addition, the side wall 112 of the first groove 11 can guide the motion of the separator 42, so that tilting and unsmooth movement can be avoided, and the valve plug component 4 moves more smoothly. This helps to perform optimization, and solve or avoid problems such as freezing of the solenoid valve, poor attachment of the separator, slow air release, and air leakage of the solenoid valve. In addition, when the magnetic member 32 is used as the reset component, an outer circumference of the protrusion 212 may be closely attached (for example, connected) to the side wall 112 of the first groove 11, or a small gap is disposed between the outer circumference of the protrusion 212 and the side wall 112 of the first groove 11. In this way, a size of the solenoid valve can be reduced, and miniaturization of the solenoid valve can be achieved.

In some embodiments, when the outer circumference of the protrusion 212 is connected to the side wall 112 of the first groove 11, no step may be disposed between the first body 211 and the protrusion 212. Correspondingly, an outer circumference of the first body 211 is flush with the outer circumference of the protrusion 212.

In some embodiments, when the valve plug component 4 includes the sealing member 41 and the separator 42, the magnetic member 32 is located above the separator 42. In other words, projection of the magnetic member 32 in the fastening direction of the housing 1 and the magnetic yoke 21 at least partially overlaps projection of the separator 42 in the fastening direction of the housing 1 and the magnetic yoke 21. In this way, the action of the magnetic force of the magnetic member 32 can effectively take effect, so that the separator 42 is more evenly supported by the force and moves more stably, and blocking effect of the sealing member 41 can be improved.

In some embodiments, the magnetic member 32 includes a magnetic material such as a permanent magnet material or a soft magnetic material. In this embodiment of this application, to enable the magnetic member 32 to attract the valve plug component 4 (specifically, the separator 42), at least one of the magnetic member 32 and the valve plug component 4 includes the permanent magnet material. For example, the magnetic member 32 is a permanent magnet, and the separator 42 is a soft magnet; or the magnetic member 32 is a soft magnet, and the separator 42 is a permanent magnet; or both the magnetic member 32 and the separator 42 are permanent magnets.

In some embodiments, at least one second accommodation space 83 may be disposed on the housing 1, the electromagnetic component 3 may include at least one magnetic member 32, and the at least one second accommodation space 83 one-to-one corresponds to the at least one magnetic member 32. Specifically, one of the at least one second accommodation space 83 is configured to accommodate one of the at least one magnetic member 32.

For example, a plurality of second accommodation spaces 83 may be disposed on the housing 1, and the plurality of second accommodation spaces 83 may be arranged in an annular array (for example, a circular ring, a square ring, or a triangular ring). Correspondingly, magnetic members 32 accommodated in the plurality of second accommodation spaces 83 may form a magnet array.

For another example, one second accommodation space 83 may be disposed on the housing 1. For example, the second accommodation space 83 may be a ring (for example, a circular ring, a square ring, or a triangular ring), and the magnetic member 32 accommodated in the second accommodation space 83 may be a ring magnet. The integrated ring magnet is easy to mount and can improve assembly efficiency of the solenoid valve.

For still another example, the reset component 3 may be disposed on the side that is of the valve plug component 4 and that faces the electromagnetic component 2 and the side that is of the valve plug component 4 and that is away from the electromagnetic component 2, that is, the reset component 3 may be disposed on two sides of the valve plug component 4. For example, with reference to FIG. 4 to FIG. 6 and FIG. 7 to FIG. 9, the reset component 3 may include the elastic member 31 and the magnetic member 32. The elastic member 31 and the coil 22 are disposed on the same side of the valve plug component 4, and the magnetic member 32 and the coil 22 are disposed on different sides of the valve plug component 4. Correspondingly, for the operating process of the solenoid valve, refer to the foregoing related descriptions about FIG. 4 to FIG. 9. For brevity, details are not described herein again.

When the reset component 3 includes both the elastic member 31 and the magnetic member 32, double insurance may be provided for reset of the valve plug component 4, and a failure rate of the reset component 3 can be reduced.

In this embodiment of this application, one of the first medium flow channel 71 and the second medium flow channel 72 is an inlet flow channel of the solenoid valve, and the other is an outlet flow channel. When the operating medium is gas, one of the first medium flow channel 71 and the second medium flow channel 72 is an air inlet flow channel, and the other is an air outlet flow channel. When the operating medium is liquid, one of the first medium flow channel 71 and the second medium flow channel 72 is a liquid inlet flow channel, and the other is a liquid outlet flow channel.

In some embodiments, refer to FIG. 4 to FIG. 9. The solenoid valve according to embodiments of this application, for example, the solenoid valve 500 or the solenoid valve 600, may further include an electrical connector 5. The electrical connector 5 is configured to electrically connect to the coil 22, to provide an electrical signal for the coil 22.

In some embodiments, the electrical connector 5 may be fastened to the outer wall (for example, the second outer wall 142) of the housing 1.

In some embodiments, FIG. 9 is used as an example. A cable hole 84 communicating with the first accommodation space 213 may be disposed on the magnetic yoke 21, and the cable hole 84 is configured to electrically connect the electrical connector 5 to the coil 22 accommodated in the first accommodation space 213. For example, a lead of the coil may be threaded out of the cable hole 84 and welded to the electrical connector 5.

In some embodiments, the cable hole 84 may be blocked through adhesive dispensing, a double-sided tape, a deformed material, or the like to ensure sealability of the solenoid valve.

In some embodiments, refer to FIG. 4 to FIG. 9. The solenoid valve according to embodiments of this application, for example, the solenoid valve 500 or the solenoid valve 600, may further include a dustproof component 6. When the size of the solenoid valve is miniaturized, the solenoid valve is more sensitive to particulate matter, and the dustproof component 6 can prevent foreign objects such as dust from entering the solenoid valve, to avoid affecting a normal function of the solenoid valve.

In some embodiments, the dustproof component 6 may include a mesh object with micropores, to block the dust. Herein, the operating medium of the solenoid valve may penetrate the dustproof component 6.

In some embodiments, the dustproof component 6 may be disposed at the inlet flow channel and/or the outlet flow channel of the solenoid valve. For example, refer to FIG. 5, FIG. 6,

FIG. 8, or FIG. 9, the first medium flow channel 71 may be the outlet flow channel, and the dustproof component 6 may be disposed at an opening, namely, the second opening 712, that is of the first medium flow channel 71 and that communicates with the outside of the housing 1. The second medium flow channel 72 is the inlet flow channel, and the dustproof component 6 may alternatively be disposed at an opening, for example, the fourth opening 722, that communicates the second medium flow channel 72 with an airbag. Certainly, in some other embodiments, the dustproof component 6 may alternatively be disposed in an air path between the inlet flow channel of the solenoid valve and the airbag.

In some embodiments, the dustproof component 6 may be fastened to the solenoid valve (which may be specifically the housing 1) through bonding, welding, or clamping, or in another fastening manner.

In some embodiments, FIG. 9 is used as an example. A fourth groove 85 whose opening faces away from the valve plug component 4 may be disposed on the first outer wall 141 of the housing 1. The first medium flow channel 71 communicates with the fourth groove 85, and the second opening 712 is formed on the bottom wall of the fourth groove 85. The fourth groove 85 is configured to accommodate the dustproof component 6. In this way, the dustproof component 6 is hidden in the housing 1, so that the thickness of the solenoid valve in the direction in which the housing 1 is fastened to the magnetic yoke 21 can be reduced, thereby helping achieve the miniaturization of the solenoid valve. Similarly, the fourth groove 85 may alternatively be disposed on a side that is of the first body 211 and that is away from the protrusion 212, to mount the dustproof component at the second medium flow channel 72.

The foregoing describes structures of the solenoid valves according to embodiments of this application with reference to FIG. 3 to FIG. 9. In this embodiment of this application, because components of the solenoid valve are simplified, an overall size of the solenoid valve can be reduced. By way of example rather than limitation, as shown in FIG. 3, the solenoid valve according to embodiments of this application may be approximately a cylinder, a minimum diameter of the solenoid valve may be from 4 mm to 8 mm, for example, 4.5 mm, 5 mm, 6 mm, or 7.5 mm, and a minimum height of the solenoid valve may be from 2 mm to 4 mm, for example, 3 mm or 3.5 mm. The miniaturization of the solenoid valve can reduce space occupied by the solenoid valve in an electronic device, and helps achieve miniaturization of the electronic device. However, it may be understood that a shape of the solenoid valve shown in FIG. 3 is merely an example. An appearance shape of the solenoid valve may alternatively be designed as another shape based on an actual requirement, for example, a square. This is not limited in embodiments of this application.

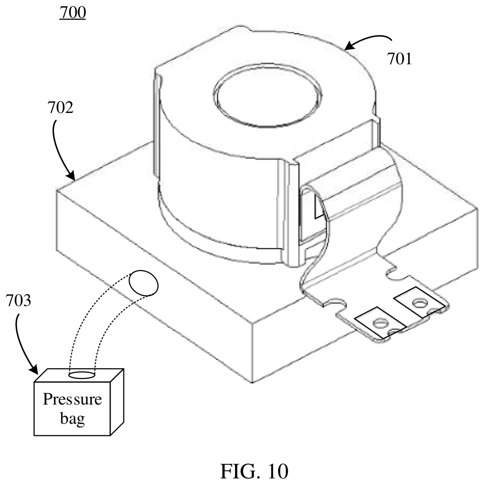

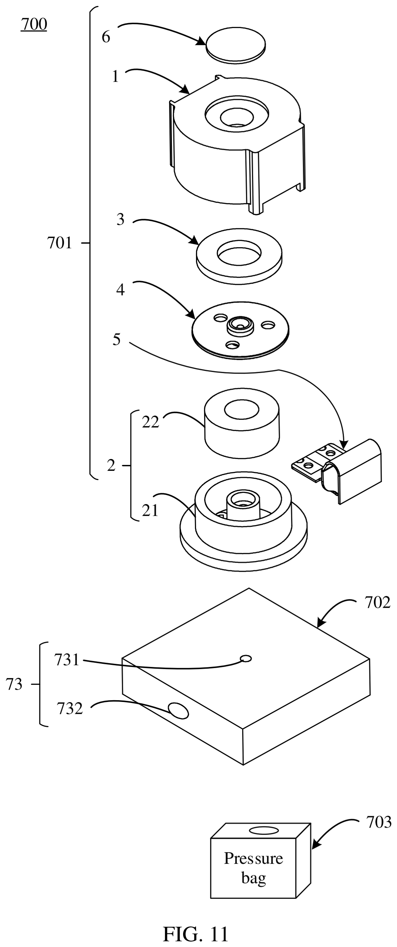

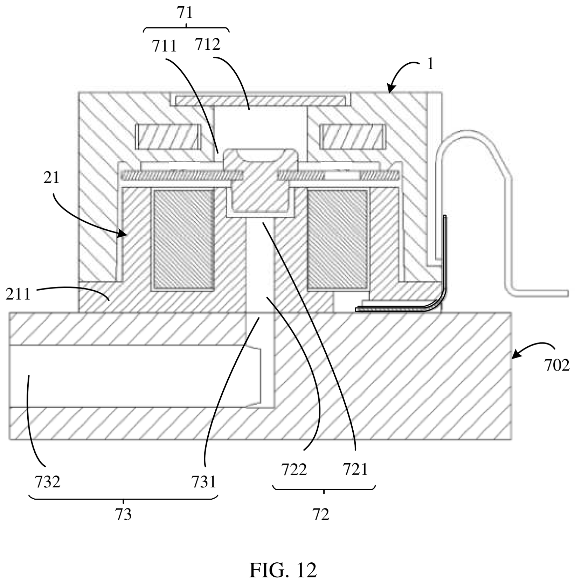

FIG. 10 to FIG. 12 are diagrams of structures of a component for pressurization and pressure relief according to embodiments of this application. FIG. 10 is an assembly diagram of the component for pressurization and pressure relief according to an embodiment of this application, FIG. 11 is an exploded diagram of the component for pressurization and pressure relief according to an embodiment of this application, and FIG. 12 is a cross-sectional diagram of the component for pressurization and pressure relief according to an embodiment of this application.

Refer to FIG. 10 to FIG. 12. The component 700 for pressurization and pressure relief may include a solenoid valve 701, a substrate 702, and a pressure bag 703. The solenoid valve 701 is connected to the substrate 702, and the substrate 702 is configured to communicate a flow channel between the pressure bag 703 and the solenoid valve 701. In this embodiment of this application, one of the first medium flow channel 71 and the second medium flow channel 72 is an inlet flow channel of the solenoid valve 701, and the other is an outlet flow channel of the solenoid valve 701. The substrate 702 is connected to a component on which the inlet flow channel (that is, a non-outlet flow channel) of the solenoid valve 701 is located. For example, the following uses an example in which the second medium flow channel 72 is the inlet flow channel of the solenoid valve 701 and the first medium flow channel 71 is the outlet flow channel of the solenoid valve 701. Correspondingly, the substrate 702 is connected to the magnetic yoke 21 of the solenoid valve 701.

The solenoid valve 701 may be the solenoid valve 500 or the solenoid valve 600 described above. For description of the solenoid valve 701, refer to related descriptions of the solenoid valve 500 or the solenoid valve 600. For brevity, details are not described herein again.

Refer to FIG. 11 or FIG. 12. A third medium flow channel 73 is disposed on the substrate 702. The third medium flow channel 73 includes a fifth opening 731 and a sixth opening 732. The fifth opening 731 is disposed on a surface that is of the substrate 702 and that faces the magnetic yoke 21, the fifth opening 731 is opposite to the fourth opening 722 of the second medium flow channel 72, and the sixth opening 732 is disposed on a surface of the substrate 702 other than a surface connected to the magnetic yoke 21. The third medium flow channel 73 is located inside the substrate 702, that is, a single component, namely, the substrate 702, can form the complete third medium flow channel 73, so that sealability of a pipe can be improved, and assembly can be facilitated. In addition, the first medium flow channel 71, the second medium flow channel 72, and the third medium flow channel 73 are all flow channels disposed inside the components, and their paths can be designed based on an actual requirement, eliminating the need for piping layout via a sleeve at the solenoid valve 701, so that the solenoid valve 701 and the entire component for pressurization and pressure relief can have a more uniform shape, and an overall size can be reduced, to be applicable to an extreme space requirement of an electronic device such as a wearable product.

In this embodiment of this application, the third medium flow channel 73 (specifically, the sixth opening 732) of the substrate 702 may be directly or indirectly connected to an outlet of the pressure bag 703. When the third medium flow channel 73 is indirectly connected to the outlet of the pressure bag 703, another component having a medium flow channel may be further disposed between the substrate 702 and the pressure bag 703, to communicate the third medium flow channel 73 with the pressure bag. This is not limited in this embodiment of this application.

A connection between the substrate 702 and the solenoid valve 701 may be a detachable connection, or may be a non-removable connection. This is not limited in this embodiment of this application. For example, the substrate 702 may be fastened to the solenoid valve 701 through bonding, welding, clamping, a thread connection, or the like. In some embodiments, the substrate 702 may alternatively be used as a part of the solenoid valve 701. To be specific, the solenoid valve 701 may further include the substrate 702, and the third medium flow channel 73 disposed on the substrate 702 communicates with the non-outlet flow channel of the first medium flow channel 71 and the second medium flow channel 72.

In this embodiment of this application, when the substrate 702 is connected to the magnetic yoke 21, because the magnetic yoke is made of a metal material, a surface that is of the magnetic yoke 21 and that is connected to the substrate 702 may have good flatness, so that sealability can be easily ensured during the connection of the substrate 702 and the magnetic yoke 21.

In some embodiments, the pressure bag 703 may be filled with gas. In this case, the pressure bag 703 is an airbag. The component 700 for pressurization and pressure relief according to this embodiment of this application may be used in the electronic sphygmomanometer, that is, the electronic device 410 or 420 shown in FIG. 2. With reference to FIG. 2 and the related corresponding accompanying drawings, a specific operating process of the component 700 for pressurization and pressure relief may be as follows.

Case 1: After the coil 22 is powered on, the coil 22 of the solenoid valve is configured to block the second medium flow channel 72. Refer to FIG. 5 or FIG. 8.

When the electronic sphygmomanometer performs inflation for the entire device, for example, the user taps the measurement button on the display of the electronic sphygmomanometer or presses the measurement key of the electronic sphygmomanometer, the processor 402 controls the air pump 4011 to inflate the pressure bag 703, and controls the coil 22 to be powered on, so that the magnetic yoke 21 is magnetized and overcomes the action force of the reset component 3 to attract the valve plug component 4 to move towards the side of the coil 22. After the valve plug component 4 reaches the stable state, the valve plug component 4 blocks the third opening 721 of the second medium flow channel 72, so that an air path of the solenoid valve and an air path of the substrate 702 maintain a sealed state, and the air paths are blocked. In this case, the gas in the pressure bag 703 cannot be discharged via the solenoid valve.

When the electronic sphygmomanometer performs deflation for the entire device, for example, the processor 402 detects that measurement is completed or a measurement result is obtained, the processor 402 may control the coil 22 to be powered off, so that the magnetic force of the magnetic yoke 21 disappears. Under the action force of the reset component 3, the valve plug component 4 moves towards the side away from the coil 22. After the valve plug component 4 reaches the stable state, the valve plug component 4 does not block the third opening 721 of the second medium flow channel 72, that is, the third opening 721 is in an open state. The air path of the solenoid valve communicates with the air path of the substrate 702. The gas in the pressure bag 703 may enter the solenoid valve via the third medium flow channel 73 of the substrate 702, and flow out of the solenoid valve via the second medium flow channel 72 and the first medium flow channel 71 that are communicated.

In the case 1, the powered-on coil 22 can maintain the air path sealed during inflation, and the coil 22 is powered off to maintain the air path open in remaining time. In this way, pressure of the entire air path is balanced with external pressure, and a service life of a sealing component can be prolonged.

Case 2: When the coil 22 is not powered on, the coil 22 of the solenoid valve is configured to block the first medium flow channel 71. Refer to FIG. 6 or FIG. 9.

When the electronic sphygmomanometer performs inflation for the entire device, for example, the user taps the measurement button on the display of the electronic sphygmomanometer or presses the measurement key of the electronic sphygmomanometer, the processor 402 controls the air pump 4011 to inflate the pressure bag 703, and controls the coil 22 to maintain the power-off state. Under the action force of the reset component 3, the valve plug component 4 blocks the first opening 711 of the first medium flow channel 71, so that the air path of the solenoid valve and the air path of the substrate 702 maintain a sealed state, and the air paths are blocked. In this case, the gas in the pressure bag 703 cannot be discharged via the solenoid valve.

When the electronic sphygmomanometer performs deflation for the entire device, for example, the processor 402 detects that measurement is completed or a measurement result is obtained, the processor 402 may control the coil 22 to be powered on, so that the magnetic yoke 21 is magnetized and overcomes the action force of the reset component 3 to attract the valve plug component 4 to move towards the side of the coil 22. After the valve plug component 4 reaches the stable state, the valve plug component 4 does not block the first opening 711 of the first medium flow channel 71, that is, the first opening 711 is in an open state. The air path of the solenoid valve communicates with the air path of the substrate 702. The gas in the pressure bag 703 may enter the solenoid valve via the third medium flow channel 73 of the substrate 702, and flow out of the solenoid valve via the second medium flow channel 72 and the first medium flow channel 71 that are communicated.

When blood pressure is measured, inflation time is usually greater than deflation time. In the case 2, the coil 22 is powered on only when deflation is performed. In an inflation process, sealing of the air path can be ensured without maintaining power always on. Therefore, energy consumption can be reduced.

It may be understood that, in the case 1 and the case 2, when the pressure bag 703 is deflated, the processor 402 may further control the air pump 4011 to deflate the pressure bag 703.

It should be noted that, as mentioned in the foregoing embodiments, the first medium flow channel 71 includes the first opening 711 and the second opening 712, the second medium flow channel 72 includes the third opening 721 and the fourth opening 722, and the third medium flow channel 73 includes the fifth opening 731 and the sixth opening 732. For example, an operating medium flows from the third medium flow channel 73 to the first medium flow channel 71 via the second medium flow channel 72, and flows out of the first medium flow channel 71.

For the third medium flow channel 73, the sixth opening 732 and the fifth opening 731 are respectively an inlet and an outlet of the third medium flow channel 73.

For the second medium flow channel 72, the fourth opening 722 and the third opening 721 are respectively an inlet and an outlet of the second medium flow channel 72.

For the first medium flow channel 71, the first opening 711 and the second opening 712 are respectively an inlet and an outlet of the first medium flow channel 71.

For the entire solenoid valve, the second medium flow channel 72 and the first medium flow channel 71 are respectively the inlet flow channel and the outlet flow channel of the solenoid valve. Correspondingly, the fourth opening 722 and the third opening 721 are inlets of the flow channel of the solenoid valve, and the first opening 711 and the second opening 712 are outlets of the flow channel of the solenoid valve.

In addition, the coil 22 in embodiments of this application should be understood in a broad sense. The coil 22 may be a winding element, that is, a basic unit that forms a winding, and is mainly formed by winding one or more turns of insulated conducting wires based on a specific shape; or may be a coil group, that is, formed by connecting a plurality of winding elements; or may be a winding, that is, formed by connecting a plurality of winding elements or coil groups according to a specific rule.

An embodiment of this application further provides a wearable device. The wearable device may include the solenoid valve (for example, the solenoid valve 500 or the solenoid valve 600) or the component 700 for pressurization and pressure relief in the foregoing embodiments.

In some embodiments, the wearable device may further include a binding band, and the binding band is configured to bind the pressure bag 703 to a body part of the user. For example, the body part of the user includes any one of a wrist, an arm, and an ankle.

In some embodiments, the pressure bag 703 is packaged in the binding band.

In some embodiments, the wearable device may further include a pump, and the pump is configured to pressurize the pressure bag 703. For example, the pump may be an air pump or a liquid pump.

In some embodiments, the wearable device may further include a pressure sensor, and the pressure sensor is configured to detect pressure of the pressure bag 703.

**In** some embodiments, the wearable device may be configured to measure blood pressure of the user, for example, may be an electronic sphygmomanometer. More specifically, the wearable device may be a blood pressure watch.

The foregoing descriptions are merely specific implementations of this application, but are not intended to limit the protection scope of this application. Any variation or replacement readily figured out by a person skilled in the art within the technical scope disclosed in this application shall fall within the protection scope of this application. Therefore, the protection scope of this application shall be subject to the protection scope of the claims.

## Claims

1. A solenoid valve, comprising:
a housing, wherein a first medium flow channel is disposed on the housing;
an electromagnetic component, comprising a magnetic yoke and a coil, wherein a second medium flow channel and a first accommodation space that are separated from each other are disposed on the magnetic yoke, the coil is accommodated in the first accommodation space, and the electromagnetic component and the housing are fastened to form an accommodation cavity;
a valve plug component, accommodated in the accommodation cavity, wherein the valve plug component is disposed between the electromagnetic component and the housing; and
a reset component, accommodated in the accommodation cavity, wherein the reset component is disposed on a side that is of the valve plug component and that faces the electromagnetic component and/or a side that is of the valve plug component and that is away from the electromagnetic component, wherein
the electromagnetic component is configured to drive the valve plug component to move when the coil is powered on, and the reset component is configured to drive the valve plug component to move when the coil is powered off, to enable the first medium flow channel to communicate with or be isolated from the second medium flow channel.

2. The solenoid valve according to claim 1, wherein
a first groove is disposed on a side that is of the housing and that faces the magnetic yoke; and
the magnetic yoke comprises a first body and a protrusion protruding from a surface of the first body, the first body is connected to the housing, the first accommodation space is disposed on the protrusion, and the protrusion is accommodated in the first groove.

3. The solenoid valve according to claim 2, wherein
the first medium flow channel communicates with the first groove; and
the second medium flow channel communicates with the first groove.

4. The solenoid valve according to claim 2 or 3, wherein
the first medium flow channel comprises a first opening and a second opening, the first opening is disposed on a bottom wall or a side wall of the first groove, and the second opening is disposed on an outer wall of the housing; and/or
the second medium flow channel comprises a third opening and a fourth opening, the third opening is disposed on a surface that is of the protrusion and that faces the bottom wall of the first groove, and the fourth opening is disposed on a surface that is of the first body and that is away from the bottom wall of the first groove.

5. The solenoid valve according to any one of claims 2 to 4, wherein the protrusion comprises a first protrusion and a second protrusion, the second protrusion is disposed around the first protrusion, and the first accommodation space is formed between the first protrusion and the second protrusion.

6. The solenoid valve according to claim 5, wherein an opening that is of the second medium flow channel and that is close to the valve plug component is disposed on a surface that is of the first protrusion and that faces the bottom wall of the first groove.

7. The solenoid valve according to any one of claims 2 to 6, wherein the reset component comprises an elastic member, the elastic member is sleeved on the protrusion, one end of the elastic member presses against the valve plug component, and the other end of the elastic member presses against the first body.

8. The solenoid valve according to claim 7, wherein the elastic member is any one of a spring, a corrugated pipe, and an elastic block.

9. The solenoid valve according to claim 7 or 8, wherein
when the coil is powered on, the magnetic yoke attracts the valve plug component, the elastic member is in a compressed state, and the valve plug component abuts against the protrusion under action of a magnetic force of the magnetic yoke and an elastic force of the elastic member.

10. The solenoid valve according to any one of claims 2 to 9, wherein the reset component comprises a magnetic member, the magnetic member is accommodated in a second accommodation space disposed on the housing, the second accommodation space is close to the bottom wall of the first groove and corresponds to a location of the valve plug component, the magnetic member and the coil are respectively disposed on two sides of the valve plug component, and a magnetic attraction force exists between the magnetic member and the valve plug component.

11. The solenoid valve according to claim 10, wherein the magnetic member is injected into the housing by using an insert molding process.

12. The solenoid valve according to claim 10 or 11, wherein
when the coil is powered on, the magnetic yoke attracts the valve plug component, and the valve plug component abuts against the protrusion under action of the magnetic force of the magnetic yoke and a magnetic force of the magnetic member.

13. The solenoid valve according to any one of claims 2 to 12, wherein
when the coil is powered on, the valve plug component blocks the second medium flow channel, and when the coil is not powered on, the first medium flow channel communicates with the second medium flow channel via the first groove; or
when the coil is not powered on, the valve plug component blocks the first medium flow channel, and when the coil is powered on, the first medium flow channel communicates with the second medium flow channel via the first groove.

14. The solenoid valve according to any one of claims 2 to 13, wherein the valve plug component comprises a sealing member and a separator, and the separator is configured to drive, under driving of the electromagnetic component or the reset component, the sealing member to move, to block the first medium flow channel or the second medium flow channel.

15. The solenoid valve according to claim 14, wherein
an end that is of the sealing member and that is close to the first medium flow channel protrudes towards the first medium flow channel relative to the separator, and/or
an end that is of the sealing member and that is close to the second medium flow channel protrudes towards the second medium flow channel relative to the separator.

16. The solenoid valve according to claim 15, wherein
a second groove that communicates with the second medium flow channel is disposed on a side that is of the protrusion and that faces the bottom wall of the first groove, and the second groove is configured to accommodate a part that is of the sealing member and that protrudes towards the second medium flow channel relative to the separator; and/or
a third groove that communicates with the first medium flow channel is disposed on the bottom wall of the first groove, and the third groove is configured to accommodate a part that is of the sealing member and that protrudes towards the first medium flow channel relative to the separator.

17. The solenoid valve according to any one of claims 14 to 16, wherein the separator comprises a permanent magnet material or a soft magnetic material.

18. The solenoid valve according to any one of claims 14 to 17, wherein at least one through hole is disposed on the separator, and the through hole is configured to communicate the first medium flow channel with the second medium flow channel.

19. The solenoid valve according to claim 18, wherein
a protruding part is disposed on the bottom wall of the first groove or on a surface that is of the separator and that faces the bottom wall of the first groove, the separator and the bottom wall of the first groove abut against each other via the protruding part, the protruding part and the through hole are disposed in a staggered manner, and a gap is formed in a region in which the protruding part is not disposed between the bottom wall of the first groove and the separator.

20. The solenoid valve according to any one of claims 1 to 19, wherein the first accommodation space is disposed around the second medium flow channel.

21. The solenoid valve according to any one of claims 1 to 20, wherein the solenoid valve further comprises a dustproof component, and the dustproof component is disposed on the first medium flow channel and/or the second medium flow channel of the solenoid valve.

22. The solenoid valve according to any one of claims 1 to 21, wherein the solenoid valve further comprises an electrical connector, the electrical connector is disposed on the outer wall of the housing, and the electrical connector is electrically connected to the coil via a cable hole disposed on the magnetic yoke.

23. The solenoid valve according to any one of claims 1 to 22, wherein the housing is of an integral structure, and/or the magnetic yoke is of an integral structure.

24. The solenoid valve according to any one of claims 1 to 23, wherein the housing is in sealing connection to the magnetic yoke.

25. The solenoid valve according to any one of claims 1 to 24, wherein the solenoid valve further comprises a substrate, the substrate is connected to the housing or the magnetic yoke, a third medium flow channel is disposed on the substrate, and the third medium flow channel communicates with a non-outlet flow channel of the first medium flow channel and the second medium flow channel.

26. The solenoid valve according to any one of claims 1 to 25, wherein an operating medium of the solenoid valve is gas or liquid.

27. A wearable device, comprising an airbag and the solenoid valve according to any one of claims 1 to 26, wherein the airbag communicates with the solenoid valve;
when the airbag is inflated, the first medium flow channel of the solenoid valve is isolated from the second medium flow channel; and
when the airbag is deflated, the first medium flow channel of the solenoid valve communicates with the second medium flow channel.

28. The wearable device according to claim 27, wherein the wearable device further comprises a binding band, and the binding band is configured to bind the airbag to a body part of a user.

29. The wearable device according to claim 28, wherein the airbag is packaged in the binding band.

30. The wearable device according to claim 28 or 29, wherein the body part of the user comprises any one of a wrist, an arm, and an ankle.

31. The wearable device according to any one of claims 27 to 30, wherein the wearable device further comprises an air pump, and the air pump is configured to inflate the airbag.

32. The wearable device according to any one of claims 27 to 31, wherein the wearable device further comprises a pressure sensor, and the pressure sensor is configured to detect pressure of the airbag.
